# EUROPEAN PATENT APPLICATION

(11) **EP 4 253 415 A1**
(43) Date of publication of application: **04.10.2023**
(21) Application number: 21891214.5
(22) Date of filing: 12.11.2021
(51) Int. Cl.: C07K 16/28, A61K 39/395, A61P 35/00, C12N 15/13, C12N 15/63

(54) **ANTIBODY AND PREPARATION METHOD THEREFOR**

(30) Priority: 12.11.2020 CN 202011260418; 13.11.2020 CN 202011269292
(71) Applicant: Mabwell (Shanghai) Bioscience Co., Ltd., Shanghai 201210 (CN)
(72) Inventor: WANG, Rongjuan, Beijing 102206 (CN); WANG, Shuang, Beijing 102206 (CN); ZHANG, Chang, Beijing 102206 (CN); JIAO, Shasha, Beijing 102206 (CN); ZENG, Dadi, Beijing 102206 (CN); ZHANG, Jiao, Beijing 102206 (CN)
(74) Representative: Witte, Weller & Partner Patentanwälte mbB
(86) International application number: PCT/CN2021/130320
(87) International publication number: WO 2022/100694

(57) **Abstract**

Provided are an antibody and a preparation method therefor. The antibody is, such as a humanized anti-human CD47 monoclonal antibody or fragment thereof, and a bispecific antibody targeting PD-L1 and CD47 or an antigen-binding fragment thereof. The bispecific antibody comprises two heavy chains and two light chains, variable regions of the two heavy chains are heterologous, and variable regions of the two light chains have similar sequences. The provided bispecific antibody has the light chains having the similar sequences, thus solving the technical problem of light and heavy chain mismatch during an assembly process of bispecific antibodies.

## Description

### CROSS REFERENCE TO RELATED APPLICATIONS

This application claims the priority of Chinese Patent Application 202011260418.8, filed with the China National Intellectual Property Administration on November 12, 2020, and Chinese Patent Application 202011269292.0, filed with the China National Intellectual Property Administration on November 13, 2020, which are hereby incorporated by reference in entirety.

### FIELD

The present disclosure belongs to the field of antibody engineering, particularly to an antibody and a preparation method therefor, more particularly, to a humanized anti-human CD47 monoclonal antibody or a fragment thereof, a multispecific antibody, a bispecific antibody targeting PD-L1 and CD47 or an antigen-binding fragment thereof, a polynucleotide, a vector, a host cell, a method for preparing an antibody or an antigen-binding fragment thereof, a composition, and uses thereof.

### BACKGROUND

CD47 is highly expressed in most tumor cells, and may be used as a standard for tumor diagnosis and prognosis. For example, CD47 is overexpressed in various solid tumor cells of acute and chronic myeloid leukemia, acute lymphoblastic leukemia, non-Hodgkin's lymphoma, bladder cancer, ovarian cancer, breast cancer, rectal cancer, prostate cancer, kidney cancer, multiple myeloma and the like, or tumor stem cells such as leukemia stem cells (LSC), which is associated with poor clinical prognosis. Studies have shown that CD47 on the surface of tumor cells binds to SIRPα on the surface of immune cells such as macrophages and DC cells, activates the negative regulatory effect of SHP-1, and inhibits the migration and phagocytosis of macrophages. This is one of the main reasons for the immune escape of tumor cells with high expression of CD47. Blocking the interaction between CD47 on the surface of tumor cells and SIRP on the surface of macrophages by antibodies or other blocking agents can promote the phagocytosis of tumor cells by macrophages and DC cells, further increase the presentation of tumor antigens, activate adaptive immune response, and provide a new idea for the treatment of tumors. Currently, there are multiple therapeutic antibodies targeting CD47 in clinical trials, wherein the representative one is Hu5F9. Although therapeutic antibodies targeting CD47 have shown good therapeutic effects in hematological tumors, they have not progressed well in solid tumors. In addition, therapeutic antibodies targeting CD47 have strong red blood cells and platelet toxicity due to the wide expression of CD47 on the surface of red blood cells and platelets, and a short half-life due to antigen deposition, so that a large administration amount is needed.

PD-L1, with a full name of programmed death ligand-1, can bind to the receptor PD-1 on the surface of T cells and exert an immunosuppressive effect. PD-L1 is an inhibitory immune checkpoint molecule expressed on the surface of cells of various malignant tumor such as melanoma, non-small cell lung cancer, renal cell carcinoma, and head and neck squamous cell carcinoma. The biding of PD-L1 to the immunosuppressive receptor PD-1 on the surface of T cells can induce apoptosis, dysfunction, and depletion of T cells, thereby inhibiting the activation, proliferation, and anti-tumor functions of tumor antigen-specific T cells, and achieving immune escape of tumors. Antibodies blocking PD-1/PD-L1 can relieve the immunosuppressive effect of PD-L1, enhance the recognition and killing of tumor cells by immune cells T cells in the body, so as to achieve the effect of killing tumors. At present, a number of antibody drugs targeting PD-1/PD-L1 have been marketed worldwide, and have shown good therapeutic effects clinically on various tumors such as melanoma, lung cancer, kidney cancer, Hodgkin's lymphoma, head and neck squamous cell carcinoma, and urothelial carcinoma. At present, despite certain clinical effects, therapeutic antibodies against PD-1/PD-L1 have low efficiency in clinical use. For most cancer types, if the antibodies are used alone indiscriminately, the average efficiency is only 10%-20%. Therefore, it is necessary to develop more effective antibody drug molecules to meet clinical needs.

In the tumor microenvironment, cancer cells may avoid the recognition and attack of the human immune system by up-regulating the expression level of PD-L1. CD47 protein, an immunoregulatory molecule overexpressed on cancer cells, mainly inhibits the phagocytosis of macrophages by interacting with inhibitory receptor signal regulatory protein α (SIRPα), and mediates the immune escape of various malignant tumors. The bispecific antibody targeting PD-L1/CD47 can simultaneously block the two pathways of CD47 and PD-L1, and is expected to provide stronger anti-tumor activity, thereby solving the problem of low efficiency of using PD-L1 antibody alone clinically. At present, researchers attempt to use anti-PD-L1 antibody in combination with anti-CD47 antibody in clinical, expecting to superimpose the immunomodulatory effects of the two to obtain better therapeutic effect. Moreover, more researchers are trying to construct bispecific antibodies targeting PD-L1/CD47, which can simultaneously block the two pathways of CD47 and PD-L1, so as to provide stronger anti-tumor activity, thereby solving the problem of low efficiency of using PD-L1 antibodies alone clinically.

Although the current PD-L1/CD47 bispecific antibodies have made some progress in the treatment of tumors, and have overcome the defects of using anti-PD-L1 and anti-CD47 alone to a certain extent, and the following defects are still existing: 1. In terms of the selection of anti-CD47 molecules, SIRPα protein is mainly selected as the binding target instead of antibody molecules. There is a significant gap between such antibody-like recombinant proteins and the monoclonal antibodies in terms of stability, quality, and PK/PD characteristics. 2. The difference in the sequences of the two chains A and B in the antibody brings great difficulties to the expression, purification process and quality control, resulting in high mispairing rate of the light chain and heavy chain, and difficulties in purification and low recovery rate of correctly assembled bispecific antibody molecules. 3. PD-L1/CD47 bispecific antibodies still have the possibility of binding to red blood cells to cause hemolysis and agglutination, so that the ability thereof of binding to red blood cells needs to be further reduced.

Therefore, anti-CD47 humanized antibodies and PD-L1/CD47 bispecific antibodies need to be further developed.

### SUMMARY

In order to solve the above problems, the present disclosure provides an anti-CD47 humanized antibody and a PD-L1/CD47 bispecific antibody. The anti-CD47 humanized antibodies bind to human CD47 and block the binding of CD47 and SIRPα. Besides, the antibody is an anti-CD47 antibody with medium or low affinity, and only specifically binds to CD47 on the surface of tumor cells, but does not bind to CD47 on the surface of human red blood cells, thereby solving the problems of hemolysis and agglutination caused by antibody binding to red blood cells. The bispecific antibody comprises two heavy chains and two light chains, wherein the variable regions of the two heavy chains are heterologous, and variable regions of the two light chains have the same sequence. In particular, in the bispecific antibody targeting CD47 and PD-L1, the variable regions of the two heavy chains are derived from a heavy chain variable region of an anti-CD47 monoclonal antibody and a heavy chain variable region of an anti-PD-L1 monoclonal antibody, respectively; and the two light chains with the same sequence are obtained from the optimization design based on the sequence similarity of the light chain of the anti-CD47 monoclonal antibody and the light chain of the anti-PD-L1 monoclonal antibody. The bispecific antibody provided by the present disclosure has light chains with the same sequence, effectively solving the technical problem of mispairing light chain and heavy chain in the assembly process of bispecific antibodies.

### Anti-CD47 antibody

In the embodiment of the present disclosure, a murine antibody that specifically binds to human CD47 is prepared. Based on the sequence analysis of the murine antibody, a similar human antibody germline template is selected to prepare a humanized anti-human CD47 monoclonal antibody or antigen-binding fragment thereof. Provided is a humanized anti-human CD47 monoclonal antibody or antigen-binding fragment thereof, which has the activity of blocking the binding of SIRPα and CD47 on the cell surface and the activity of tumor suppression. Furthermore, the humanized anti-human CD47 monoclonal antibody is subjected to point mutation modification in CDRs and FRs to adjust the affinity for human CD47, and the light chain is further mutated to generate antibodies with different binding activity to CD47 on the surface of tumor cells and to CD47 on the surface of human red blood cells, so as to obtain a series of anti-CD47 antibodies without red blood cells binding activity. Further, a humanized anti-human CD47 monoclonal antibody that can tolerate multiple amino acid site mutations in the light chain CDRs while maintaining the ability of specifically binding to CD47 is selected, thereby providing a new platform for the development of bispecific antibodies with the same light chain.

According to one aspect of the present disclosure, the present disclosure provides a humanized anti-human CD47 monoclonal antibody or a fragment thereof. According to an embodiment of the present disclosure, the humanized anti-human CD47 monoclonal antibody or fragment thereof comprises a heavy chain CDR1 comprising X₁YX₂MX₃, wherein X₁ is selected from the group consisting of N and S, X₂ is selected from the group consisting of V and A, and X₃ is selected from the group consisting of H and S; a heavy chain CDR2 comprising YINPX₄NX₅X₆IKYNEKFX₇G, wherein X₄ is selected from the group consisting of Y and G, X₅ is selected from the group consisting of D and E, X₆ is selected from the group consisting of G and A, and X₇ is selected from the group consisting of T and Q; and a heavy chain CDR3 comprising EGDFYANYGRLGFX₈Y, wherein X₈ is selected from the group consisting of A and D; and a light chain CDR1 comprising RASQDIX₉NYLN, wherein X₉ is selected from the group consisting of S and T; a light chain CDR2 comprising YTSRLX₁₀S, wherein X₁₀ is selected from the group consisting of H, Q and S; and a light chain CDR3 comprising QQGX₁₁X₁₂X₁₃PX₁₄T, wherein X₁₁ is selected from the group consisting of D and A, X₁₂ is selected from the group consisting of T and G, X₁₃ is selected from the group consisting of F, R, Y, K, S, E and N, and X₁₄ is selected from the group consisting of Y and R.

According to an embodiment of the present disclosure, the humanized anti-human CD47 monoclonal antibody or antigen-binding fragment thereof is derived from a germline template selected from the group consisting of IGKV1-33^{∗}01|IGKJ4^{∗}02 and IGHV1-3^{∗}01|IGHJ1^{∗}01.

According to an embodiment of the present disclosure, the CDRs and/or FRs of the humanized anti-human CD47 monoclonal antibody or fragment thereof comprise a point mutation, which help to reduce or eliminate the activity of binding to red blood cells, and at least partially maintain the binding ability of the humanized anti-human CD47 monoclonal antibody or antigen-binding fragment thereof to tumor cells.

According to an embodiment of the present disclosure, the heavy chain CDR1 comprises a sequence set forth in SEQ ID NO: 76, 77, 78 or 87; the heavy chain CDR2 comprises a sequence set forth in SEQ ID NO: 79, 80, 81, 82, 83 or 84; and the heavy chain CDR3 comprises a sequence set forth in SEQ ID NO: 85 or 86.

According to an embodiment of the present disclosure, the light chain CDR1 comprises a sequence set forth in SEQ ID NO: 46 or 47; the light chain CDR2 comprises a sequence set forth in SEQ ID NO: 50, 51 or 52; and the light chain CDR3 comprises a sequence set forth in SEQ ID NO: 56, 57, 58, 59, 60, 61, 62, 63, 64, 65 or 68.

According to an embodiment of the present disclosure, the humanized anti-human CD47 monoclonal antibody or fragment thereof comprises a heavy chain variable region set forth in SEQ ID NO: 8, 11, 12, 13, 14, 15, 16, 17, 18, 19, 20, 21, 22 or 23, and a light chain variable region set forth in SEQ ID NO: 5.

According to an embodiment of the present disclosure, the monoclonal antibody of the humanized anti-human CD47 monoclonal antibody or fragment thereof is an antibody derived from an original monoclonal antibody by CDRs transplantation, affinity maturation, point mutation and chemical modification, wherein the chemical modification is selected from the group consisting of glycosylation, acetylation, PEGylation, phosphorylation, amidation, protease cleavage, linking with cellular ligands or effector molecules, and protection and/or blocking of an active reactive group.

Further, any one of the humanized anti-human CD47 monoclonal antibodies or fragments thereof in the present disclosure may be a Fab, Fab', F(ab')₂, Fv, scFv or dAb.

Further, according to another aspect of the present disclosure, the present disclosure provides a humanized anti-human CD47 monoclonal antibody or a fragment thereof, which is humanized from a murine anti-human CD47 monoclonal antibody by using IGKV1-33^{∗}01|IGKJ4^{∗} 02 and IGHV1-3^{∗}01|IGHJ1^{∗}01 as germline templates.

According to an embodiment of the present disclosure, the humanized anti-human CD47 monoclonal antibody or fragment thereof comprises a heavy chain variable region comprising a sequence set forth in SEQ ID NO: 8 and CDRs sequences (HCDR1, HCDR2, HCDR3), and a light chain variable region comprising a sequence set forth in SEQ ID NO: 5 and CDRs sequences (LCDR1, LCDR2, LCDR3).

According to an embodiment of the present disclosure, the humanized anti-human CD47 monoclonal antibody or fragment thereof comprises a heavy chain variable region set forth in SEQ ID NO: 8, and a light chain variable region set forth in SEQ ID NO: 5.

Further, according to an embodiment of the present disclosure, the humanized anti-human CD47 monoclonal antibody or fragment thereof comprises:
a heavy chain CDR1 comprising X₁YX₂MX₃, wherein X₁ is selected from the group consisting of N and S, X₂ is selected from the group consisting of V and A, and X₃ is selected from the group consisting of H and S;
a heavy chain CDR2 comprising YINPX₄NX₅X₆IKYNEKFX₇G, wherein X₄ is selected from the group consisting of Y and G, X₅ is selected from the group consisting of D and E, X₆ is selected from the group consisting of G and A, and X₇ is selected from the group consisting of T and Q; and
a heavy chain CDR3 comprising EGDFYANYGRLGFX₈Y, wherein X₈ is selected from the group consisting of A and D.

Further, according to an embodiment of the present disclosure, the humanized anti-human CD47 monoclonal antibody or fragment thereof comprises:
a light chain CDR1 comprising RASQDIX₉NYLN, wherein X₉ is selected from the group consisting of S and T;
a light chain CDR2 comprising YTSRLX₁₀S, wherein X₁₀ is selected from the group consisting of H, Q and S; and
a light chain CDR3 comprising QQGX₁₁X₁₂X₁₃PX₁₄T, wherein X₁₁ is selected from the group consisting of D and A, X₁₂ is selected from the group consisting of T and G, X₁₃ is selected from the group consisting of F, R, Y, K, S, E and N, and X₁₄ is selected from the group consisting of Y and R.

According to another aspect of the present disclosure, the present disclosure provides a multispecific antibody. According to an embodiment of the present disclosure, the multispecific antibody comprises at least one specific binding region for human CD47, wherein the specific binding region for human CD47 is a humanized anti-human CD47 monoclonal antibody or antigen-binding fragment thereof.

According to an embodiment of the present disclosure, the multispecific antibody comprises multiple specific binding regions for different epitopes of human CD47.

According to an embodiment of the present disclosure, the multispecific antibody further comprises a specific binding region which does not target human CD47.

According to an embodiment of the present disclosure, the multispecific antibody is a bispecific antibody, a trispecific antibody or a tetraspecific antibody.

The humanized anti-human CD47 monoclonal antibody or antigen-binding fragment thereof in the embodiment of the present disclosure has at least one of the following beneficial technical effects:
(1) The humanized anti-human CD47 monoclonal antibody or antigen-binding fragment thereof of the present disclosure can specifically bind to CD47 on the cell surface with high affinity, and effectively block the interaction between CD47 on the surface of cancer cells and SIRPa on macrophage, with a good anti-tumor effect.
(2) The humanized anti-human CD47 monoclonal antibody is subjected to point mutation modification in CDRs and FRs to adjust the affinity for human CD47, and the light chain is further mutated to differentiate the activity of the antibody binding to CD47 on the surface of tumor cells and to CD47 on the surface of human red blood cells, so as to obtain a series of anti-CD47 antibodies with high binding activity to CD47 on the surface of tumor cells and low binding activity to red blood cells, effectively solving the problem of high clinical administration amount of anti-CD47 antibodies due to the low antibody concentration caused by the binding of anti-CD47 antibodies with red blood cells and platelets clinically, and significantly reducing the toxic and side effects of anti-human CD47 antibody. In addition, the anti-human CD47 antibody provides a new molecule option for solving the problem of low blood drug concentration caused by antigen deposition.
(3) The humanized anti-human CD47 monoclonal antibody of the present disclosure can tolerate multiple amino acid site mutations in the light chain variable region while maintaining the ability of specifically binding to CD47, therefore providing a new platform for the development of bispecific antibodies with the same light chains.

### Bispecific antibodies targeting PD-L1 and CD47 or antigen-binding fragments thereof

According to one aspect of the present disclosure, the present disclosure provides a method for preparing a bispecific antibody. According to an embodiment of the present disclosure, the method comprises:
(1) selecting two monoclonal antibodies with the same light chain germline and similar sequences;
(2) constructing a co-expression hybrid antibody and determining the ability thereof of binding to a first antigen, wherein the hybrid antibody comprises a heavy chain and a light chain comprising a heavy chain variable region of the first antibody and a light chain variable region of a second antibody, respectively;
(3) optionally, performing site mutation on the hybrid antibody to construct a population of antibody hybrids, determining the ability of each antibody to bind to the first antigen, and selecting a first heavy chain therefrom, wherein the first heavy chain form a hybrid antibody with a light chain of the second antibody, which has high affinity and good specificity for the first antigen;
(4) mutating the light chain variable region of the second antibody to construct a second antibody light chain mutant population, which is co-expressed with the first heavy chain selected in step (3) to prepare a first heavy chain/second antibody mutant hybrid antibody, detecting the ability thereof of binding to the first antigen, and selecting a common light chain;
(5) selecting a second heavy chain variable region according to the heavy chain variable region of the second antibody; and
(6) co-expressing an antibody heavy chain and light chain comprising the first heavy chain variable region selected in step (3), the common light chain variable region selected in step (4), and the second heavy chain variable region selected in step (5) to produce a bispecific antibody.

According to an embodiment of the present disclosure, in the step (4), each amino acid residue in the variable region of the light chain mutant of the second antibody is at least the same as the amino acid residue at the corresponding position in the light chain variable region of the first antibody molecule, and/or the amino acid residue at the corresponding position in the light chain variable region of the second antibody molecule.

According to an embodiment of the present disclosure, the method optionally further comprises: (7) detecting the ability of the bispecific antibody specifically binding to the first antigen and the second antigen.

According to an embodiment of the present disclosure, if there is a difference between the common light chain variable region selected in step (4) and the light chain variable region of the second antibody, selecting a second heavy chain variable region according to the heavy chain variable region of the second antibody in step (5) comprises: constructing a second antibody heavy chain variable region mutant population, co-expressing the common light chain variable region therewith to prepare a second antibody heavy chain variable region mutant/common light chain variable region hybrid antibody, detecting the ability thereof of binding to the second antibody, and selecting the second heavy chain.

If the common light chain selected in step (4) is the same as the light chain of the second antibody, selecting a second heavy chain variable region according to the heavy chain variable region of the second antibody in step (5) refers to making the heavy chain of the second antibody serve as the second heavy chain.

According to another aspect of the present disclosure, the present disclosure provides a bispecific antibody or an antigen-binding fragment thereof. According to an embodiment of the present disclosure, the bispecific antibody or antigen-binding fragment thereof comprises two heavy chains and two light chains, wherein a first heavy chain variable region is derived from a heavy chain variable region of an anti-CD47 monoclonal antibody, which forms a CD47 binding region with a light chain variable region, and a second heavy chain variable region is derived from a heavy chain variable region of an anti-PD-L1 monoclonal antibody, which forms a PD-L1 binding region with a light chain variable region; wherein, the light chain variable regions of the two light chains have the same sequences, and the two light chains are selected from the group consisting of a light chain of anti-CD47 monoclonal antibody, a light chain of anti-PD-L1 monoclonal antibody, and a common light chain having a sequence between the light chain of anti-CD47 monoclonal antibody and the light chain of anti-PD-L1 monoclonal antibody, wherein the anti-CD47 monoclonal antibody is the humanized anti-human CD47 monoclonal antibody or fragment thereof of the present disclosure according to any one of claims 1-6.

According to an embodiment of the present disclosure, the light chain variable region of the first antibody molecule and the light chain variable region of the second antibody molecule belong to the same antibody light chain germline, and have the same or highly similar framework regions (FRs) .

According to an embodiment of the present disclosure, the common light chain variable region has the common amino acid residues between the light chain variable region of the first antibody molecule and the light chain variable region of the second antibody molecule. There are amino acid residues that differ between the light chain variable region of the first antibody molecule and the light chain variable region of the second antibody molecule. The common light chain is the same as the light chain of the first antibody molecule or the same as the light chain of the second antibody molecule.

According to an embodiment of the present disclosure, the first heavy chain variable region in the bispecific antibody or antigen-binding fragment thereof is derived from the heavy chain variable region of the first antibody molecule by a point mutation, and the first heavy chain variable region at least partially maintains the ability of the heavy chain variable region of the first antibody molecule forming a first antigen binding region with the light chain variable region.

According to another aspect of the present disclosure, the present disclosure further provides a bispecific antibody targeting PD-L1 and CD47 or an antigen-binding fragment thereof. According to an embodiment of the present disclosure, the bispecific antibody or antigen-binding fragment targeting PD-L1 and CD47 thereof comprises two heavy chains and two light chains, wherein a first heavy chain variable region is derived from a heavy chain variable region of an anti-CD47 monoclonal antibody, which forms a CD47 binding region with a light chain variable region, and a second heavy chain variable region is derived from a heavy chain variable region of an anti-PD-L1 monoclonal antibody, which forms a PD-L1 binding region with a light chain variable region; wherein, the light chain variable regions of the two light chains have the same sequences, and it is derived from the light chain of the anti-CD47 monoclonal antibody and the light chain of the anti-PD-L1 monoclonal antibody. The light chain variable region matches the first heavy chain variable region to form a first variable region binding region specifically binding to CD47, and the light chain variable region matches the second heavy chain variable region to form a second variable region binding region specifically binding to PD-L1, wherein the anti-CD47 monoclonal antibody is the aforementioned humanized anti-human CD47 monoclonal antibody or fragment thereof.

According to an embodiment of the present disclosure, the first variable region binding region specifically binds to CD47 with an affinity that is similar to that of the initial CD47 monoclonal antibody variable region with CD47. The second variable region binding region specifically binds to PD-L1 with an affinity that is similar to that of the initial PD-L1 monoclonal antibody variable region with PD-L1.

According to an embodiment of the present disclosure, the anti-PD-L1 monoclonal antibody comprises a heavy chain variable region set forth in SEQ ID NO: 3 and a light chain variable region set forth in SEQ ID NO: 1.

According to an embodiment of the present disclosure, the first heavy chain comprises a heavy chain variable region set forth in SEQ ID NO: 8, 11, 12, 13, 14, 15, 16, 17, 18, 19, 20, 21, 22, or 23.

According to an embodiment of the present disclosure, the light chain has a sequence set forth in SEQ ID NO: 1, 24, 25, 26, 27, 28, 29, 30, 31, 32, 33 or 34.

According to an embodiment of the present disclosure, the antibody or antigen-binding fragments thereof includes, but is not limited to, a complete antibody or F(ab')₂.

According to an embodiment of the present disclosure, the complete antibody is selected from the group consisting of IgG1 and IgG4 type antibodies.

According to an embodiment of the present disclosure, the two heavy chains comprise Fc fragments containing a mutation for "knobs-into-holes";
According to an embodiment of the present disclosure, the first heavy chain comprises an Fc fragment containing H315R mutation, the second heavy chain comprises an Fc fragment containing K319E mutation, and amino acid mutation positions in the Fc fragment are in Eu Kabat numbering system.

According to yet another aspect of the present disclosure, the present disclosure provides a polynucleotide. According to an embodiment of the present disclosure, the polynucleotide encodes the aforementioned humanized anti-human CD47 monoclonal antibody or fragment thereof, the aforementioned multispecific antibody, or the aforementioned bispecific antibody targeting PD-L1 and CD47 or antigen-binding fragment thereof.

According to yet another aspect of the present disclosure, the present disclosure provides a vector. According to an embodiment of the present disclosure, the vector comprises the aforementioned polynucleotide.

According to yet another aspect of the present disclosure, the present disclosure provides a host cell. According to an embodiment of the present disclosure, the host cell comprises the aforementioned polynucleotide or the aforementioned vector.

According to yet another aspect of the present disclosure, the present disclosure provides a method for preparing an antibody or an antigen-binding fragment thereof. According to an embodiment of the present disclosure, the method comprises the following steps: (1) culturing the aforementioned host cell according to claim 21 under a condition suitable for expressing a bispecific antibody or an antigen-binding fragment thereof; and (2) isolating and purifying the bispecific antibody or antigen-binding fragment thereof from cell culture.

Compared with the prior art, the technical solution of the bispecific antibody targeting PD-L1 and CD47 or antigen-binding fragment thereof in the embodiment of the present disclosure has at least one of the following advantages.

First, the bispecific antibody prepared in the embodiment of the present disclosure has dual binding arms structure just similar as natural antibodies, and each binding arm has a complete Fab structure, which avoids the shortcomings such as structural instability and mutual interference of functions caused by the introduction of non-antibody components such as a receptor chain (such as CD47 receptor SIRPα) into the antibody molecule. Furthermore, according to the embodiment of the present disclosure, the mutation modification of the common light chain variable region and heavy chain variable region realizes the regulation of the binding ability of the two binding arms, for example, resulting in a bispecific antibody with high capacity of binding to the first antigen/antigen epitope and moderate capacity of binding to a second antigen/epitope.

Second, according to the different impact of the six CDRs on the antigen-binding region on the antigen-binding activity in the prior art, i.e., the three CDRs of the heavy chain have a greater impact than the three CDRs of the light chain, and the impact of CDR3 is greater than that of CDR2 and CDR1, in conjunction with the similarity in light chain sequence of the anti-PD-L1 monoclonal antibody hz182 and anti-CD47 monoclonal antibody hz140, the inventors designed an anti-human PD-L1/CD47 bispecific antibody with the same light chain. The design of the common light chain not only simplifies the recombinant expression method, but also completely eliminates the problem of mispairing of light chain and heavy chain in the process of recombinant expression of a bispecific antibody, significantly simplifying the production process, and improving the qualified rate and uniformity of the product. In addition, Fc fragment is introduced with a mutation for "knobs-into-holes", which enhances the accuracy of pairing of the Fc fragment of the heavy chain of the bispecific antibody and avoids dimerization of homologous heavy chains.

Third, during the design and preparation process of the bispecific antibody, the heavy chain of the anti-CD47 antibody is subjected to limited amino acid mutations, so that both the heavy chain variable region and the light chain variable region in the anti-CD47 binding arm of the recombinant antibody are slightly different from the structure of the initial anti-CD47 monoclonal antibody. The above anti-CD47 antigen binding region is introduced with the slight difference to provide the bispecific antibody with moderate anti-CD47 activity, not only maintaining the activity of the bispecific antibody binding to CD47 on the surface of tumor cells and enhancing the tumor suppressive effect of anti-PD-L1 activity, but also significantly reducing the binding of the bispecific antibody to CD47 on the surface of red blood cells, which not only reduces or eliminates undesired consequences such as hemolysis and red blood cell agglutination, but also reduces the amount of administration.

### Applications and uses of antibodies

According to one aspect of the present disclosure, the present disclosure provides a composition. According to an embodiment of the present disclosure, the composition comprises at least one selected from the group consisting of the aforementioned humanized anti-human CD47 monoclonal antibody or fragment thereof, the aforementioned multispecific antibody, the aforementioned bispecific antibody targeting PD-L1 and CD47 or antigen-binding fragment thereof, the aforementioned polynucleotide, the aforementioned vector, and the aforementioned host cell.

According to another aspect of the present disclosure, the present disclosure provides use of the aforementioned humanized anti-human CD47 monoclonal antibody or fragment thereof, the aforementioned multispecific antibody, the aforementioned bispecific antibody targeting PD-L1 and CD47 or antigen-binding fragment thereof, the aforementioned polynucleotide and the aforementioned composition in the manufacture of a medicament for treating a tumor and/or improving immune response in the body.

According to an embodiment of the present disclosure, the tumor is selected from the group consisting of melanoma, lung cancer, kidney cancer, Hodgkin's lymphoma, head and neck squamous cell carcinoma, urothelial carcinoma, acute and chronic myeloid leukemia, acute lymphoblastic leukemia, non-Hodgkin's lymphoma, bladder cancer, ovarian cancer, breast cancer, colorectal cancer, prostate cancer, kidney cancer, and multiple myeloma.

According to yet another aspect of the present disclosure, the present disclosure provides a method for treating a subject suffering from a tumor. According to an embodiment of the present disclosure, the method comprises administering a therapeutically effective amount of the aforementioned composition to the subject in need thereof.

To better understand the present disclosure, several terms are first defined. Additional definitions are listed throughout the detailed description.

The term "PD-L1", with a full name of programmed death ligand 1, also named surface cluster of differentiation 274 (CD274) or B7 homolog 1 (B7-H1), encoded by the CD274 gene, is a ligand of PD-1 (programmed cell death receptor 1). PD-L1 is a first type of transmembrane protein with a size of 40kDa, expressed on immune cells such as T cells and B cells and tumor cells. Under normal circumstances, the immune system may respond to exogenous antigens gathered in lymph nodes or spleen, promoting the production of antigen-specific cytotoxic T cells (proliferation of CD8+ T cell). When PD-L1 on the tumor cell membrane binds to PD-1 on immune cells such as T cells, the tumor cells send out inhibitory signals, reducing the proliferation of CD8+ T cells in lymph node, further leading to failure of T cells in recognizing tumor cells and killing tumor cells, and suppressing the immune function.

The term "CD47", also named integrin-related protein, has a molecular weight of about 50kDa and is expressed in both normal cells and tumor cells. CD47 can bind to the protein SIRPα on macrophages to provide a "don't eat me" signal, thereby inhibiting the phagocytosis of macrophages and leading to immune escape of tumors. Blocking the CD47-SIRPα interaction in tumor cells, senescent red blood cells, and platelets may induce phagocytosis. On the one hand, according to this mechanism of action, CD47 has gradually become popular in tumor immunotherapy in recent years, and it is a potentially effective and widely used target for tumor immunotherapy. A number of inhibitors have been developed, which can specifically block CD47-SIRPα signaling pathway to enhance the immune response to tumors. On the other hand, however, the mechanism of blocking CD47-SIRPα interaction leading to reduction of red blood cells and platelets, which is the biggest challenge for druggability.

The term "specificity" refers to the determination of the presence or absence of the protein in a protein and/or other biologically heterogeneous population. Under specified conditions, a specific ligand/antigen binds to a specific receptor/antibody, and does not bind in a significant amount to other proteins present in the sample.

The term "antibody" herein is intended to include a full-length antibody and any antigen-binding fragment thereof (i.e., an antigen-binding moiety) or a single chain thereof. A full-length antibody is a glycoprotein comprising at least two heavy (H) chains and two light (L) chains, and the heavy chain and the light chain are linked by a disulfide bond. Each heavy chain consists of a heavy chain variable region (abbreviated as VH) and a heavy chain constant region. The heavy chain constant region consists of three domains, namely CH1, CH2 and CH3. Each light chain consists of a light chain variable region (abbreviated as VL) and a light chain constant region. The light chain constant region consists of one domain, CL. The VH and VL regions may further be divided into hypervariable regions called complementarity determining regions (CDRs), which are separated by conserved framework regions (FRs). Each VH and VL consists of three CDRs and four FRs, which are arranged in the order of FR1, CDR1, FR2, CDR2, FR3, CDR3 and FR4 from the amino terminal to the carboxyl terminal. The variable regions of the heavy and light chains comprise a binding domain that interacts with the antigen. The constant regions of the antibody can mediate the binding of the immunoglobulin to host tissues or factors, including various cells of the immune system (e.g., effector cells) and the first component of the classical complement system (Clq).

The term "monoclonal antibody" or "monoclonal antibody" or "monoclonal antibody composition" refers to a product of an antibody molecule having a single molecular composition. A monoclonal antibody composition exhibits a single binding specificity and affinity for a particular epitope.

As used herein, the "antigen-binding fragment" of an antibody (or simply referred to as an antibody moiety) refers to one or more fragments of an antibody that retain the ability to specifically bind to an antigen. It has been demonstrated that the antigen-binding function of an antibody can be exerted by a fragment of a full-length antibody. Examples of a binding fragment contained in an "antigen-binding moiety" of an antibody include (i) a Fab fragment, which is a monovalent fragment consisting of VL, VH, CL, and CH1; (ii) a F(ab')2 fragment, which is a bivalent fragment of two Fab fragments linked by sulfur bridge in the hinge region; (iii) a Fd fragment consisting of VH and CH1; (iv) a Fv fragment consisting of antibody single arms VL and VH; (v) a dAb fragment consisting of VH (Ward et al., (1989) Nature 341:544-546); (vi) an isolated complementarity determining region (CDR); and (vii) a nanoantibody, which is heavy chain variable region comprising a single variable domain and two constant domains. Furthermore, although the two domains VL and VH of the Fv fragment are encoded by different genes, they may be linked by a recombinant method via a synthetic linker that makes the two domains a single protein chain, where the VL and VH regions pair to form a monovalent molecule (named single-chain Fc (scFv); see e.g. Bird et al., (1988) Science 242:423-426; and Huston et al., (1988) Proc. Natl. Acad. Sci. USA 85:5879-5883). These single chain antibodies are also intended to be included within the meaning of the term. These antibody fragments may be obtained by common techniques known to those skilled in the art, and the fragments may be functionally screened in the same manner as complete antibodies.

The antigen-binding fragment of the present disclosure includes those capable of specifically binding to an antigen molecule. Examples of an antibody binding fragment include, but are not limited to, for example, Fab, Fab', F(ab')2, Fv fragment, single chain Fv (scFv) fragment, and a single domain fragment.

Fab fragment comprises a constant domain of a light chain and a first constant domain (CH1) of a heavy chain. Fab' fragment differs from Fab fragment by the addition of a few residues at the carboxy-terminal of the CH1 domain of the heavy chain, including one or more cysteines derived from the antibody hinge region. Fab' fragment is generated through the cleavage of a disulfide bond at the cysteines in the hinge region of a product obtained by F(ab')2 pepsin digestion. Additional chemical couplings of antibody fragments are known to those of ordinary skill in the art. Fab and F(ab')₂ fragments lack a fragment crystallizable (Fc) region of an complete antibody, which are more rapidly cleared from the circulation in animals, and may have less nonspecific tissue binding than a complete antibody (see, e.g., Wahl et al. People, 1983, J. Nucl. Med. 24:316).

"Fc" region, as generally understood in the art, is a fragment crystallizable constant region of an antibody that does not comprise an antigen-specific binding region. In IgG, IgA, and IgD antibody isotypes, Fc region consists of two identical protein fragments, derived from the second and third constant domains (CH2 and CH3 domains, respectively) of the two heavy chains of an antibody. IgM and IgE Fc regions comprise three heavy chain constant domains (CH2, CH3 and CH4 domains) in each polypeptide chain.

"Human antibody" refers to an antibody having an amino acid sequence of a human immunoglobulin, and an antibody isolated from human immunoglobulin libraries or animals, where the animals are transgenic for one or more human immunoglobulins, and do not express endogenous immunoglobulins. A human antibody may be produced by various methods known in the art, including a phage display method using antibody libraries derived from human immunoglobulin sequences. Reference may be made to US Patent Nos. 4,444,887 and 4,716,111; and PCT Publication Nos. WO 98/46645; WO 98/50433; WO 98/24893; WO 98/16654; WO 96/34096; WO96/33735 and WO 91/10741. A human antibody may also be produced using transgenic mice that do not express functional endogenous immunoglobulins, but express human immunoglobulin genes. Reference can be made to, for example, PCT Publication Nos. WO 98/24893; WO 92/01047; WO 96/34096 and WO 96/33735; US Patent Nos. 5,413,923; 5,625,126; 5,633,425; 5,569,825; 5,661,016; 5,545,806; 5,814,318; 5,885,793; 5,916,771; and 5,939,598. Alternatively, by using techniques similar to those described above, companies such as LakePharma, Inc. (Belmont, CA) or Creative BioLabs (Shirley, NY) can be engaged in providing human antibodies for the indicated antigens. A technique named "guided selection" may be used to produce a fully human antibody that recognizes the indicated epitope. In this method, the non-human monoclonal antibody selected, such as a mouse antibody, is used to guide the selection of a fully human antibody that recognizes the same epitope (see, Jespers et al., 1988, Biotechnology 12:899-903).

The term "heavy chain" refers to a heavy chain (H chain) about twice the size of a light chain, having 450-550 amino acid residues, and a molecular weight of about 55 or 75 kD. Each H chain contains a cyclic peptide consisting of 4 to 5 intrachain disulfide bonds. Different H chains have different antigenicities due to the amino acid composition and sequences, the number and position of disulfide bonds, and the types and number of amino acid contained therein. According to the difference in antigenicity of H chains, H chains may be divided into five classes: µ chain, γ chain, α chain, δ chain, and ε chain. Complete immunoglobulin molecules formed by different H chains and L chains (κ or λ chains) are named IgM, IgG, IgA, IgD, and IgE, respectively. The γ, α and δ chains contain 4 peptides, and the µ and ε chains contain 5 peptides.

The term "light chain" refers to a light chain (L chain) which is a polypeptide chain that is smaller in molecular weight compared to a heavy chain in an immunoglobulin monomer molecule. 1/2 region with variable amino acid composition and sequence near the amino terminal (N-terminal) of each light chain is a light chain variable region (VL), which is a component of an Ig molecule and an antigen-binding region. The remaining 1/2 region with relatively stable amino acid composition and sequence is a light chain constant region (CL). Due to certain differences in the amino acid sequences of the light chain constant regions, there are two types of light chains, κ and λ.

The term "germline" refers to an immunoglobulin germline. Antibody-forming cells have all the genes encoding an Ig molecule (i.e., a limited number of C genes and an unknown number of V genes), which are formed through long-term evolution and passed through germ cells from parents to offspring. The gene of the heavy chain of a human immunoglobulin is encoded by a V-D-J-C gene fragment; the gene of the light chain of a human immunoglobulin is encoded by a V-J-C gene fragment, and the number of gene fragments varies. The gene of the human heavy chain is located on the long arm of chromosome 14, spanning about 1,100 kb, consisting of four gene fragments of V, D, J and C, and comprising about 95 Va gene fragments divided into seven families of VH1∼VH7, where there are 65 functional gene fragments, 27 D gene fragments, 6 JH gene fragments and 9 CH gene fragments. Va gene fragments are located upstream, D gene fragments are located between VH and JH gene clusters, JH genes are located downstream of DH, and separated from the downstream C gene region by about 7 Kb, CH genes are arranged in cluster with a span of about 200 kb, P and S genes are located immediately downstream of the JH gene fragment, and Cy, Cα and Cε are sequentially located downstream of C8. The genes of human light chain are divided into λ and x genes, which are respectively located on the long arm of chromosome 22 and the short arm of chromosome 2. There are about 40 functional VK gene fragments, 5 functional J and 1 Cκ after Vc gene fragment, about 30 Vλ gene fragments, 4 Jλ gene fragments and 4 Cλ gene fragments.

The term "EC₅₀", also named concentration of half-maximal effect, refers to a concentration of antibody that causes 50% of the maximum effect.

The term "bispecific antibodies" refers to an antibody structure that binds to different epitopes on the same or different antigens. Therefore, a bispecific antibody is able to bridge two different molecules, playing a role to recruit effector molecules, effector cells, viruses and drug delivery systems to the target structure. A bispecific antibody can recognize two different molecules (receptors and/or ligands) simultaneously, which improves the selectivity and functional affinity of antibodies.

The term "mispair or mispairing" refers to a problem of random mispairing of light chain and heavy chain. IgG-like bispecific antibodies are usually expressed using cells. Since an antibody consists of 2 light chains (L) and 2 heavy chains (H), when they are co-expressed in one host cell, it is possible to result in 10 combinations of HHLL, while in fact, only 12.5% thereof is target heterologous antibody combination having desired functions. It is extremely challenging to purify a sufficient amount of target antibody from a complex system containing nearly 10 kinds of mixtures, which leads to the problems of low uniformity and yield of product, and complicated subsequent purification processes. The production strategy of the marketed product Catumaxomab (with an antibody type of Triomab) has been improved on this basis. For Triomab, rat hybridoma and mouse hybridoma are subjected to somatic fusion, and the species-restricted H-L chain pairing strategy can reduce the rate of random light-heavy chain mismpair (<10%). According to the different abilities of the Fc fragments of antibodies of different species and different subclasses binding to protein A medium, the target antibody with both rat Fc fragment and mouse Fc fragment may be isolated by one-step protein A affinity chromatography purification, thereby simplifying the purification steps. However, the bispecific antibody constructed based on Triomab technology is a murine antibody with strong immunogenicity, which limits the scope of the clinical application thereof.

It should be noted that the method for determining the sequence information of a DNA sample according to the embodiment of the present disclosure was completed by the inventors of the present application through hard creative effort and optimization work.

Additional aspects and advantages of the present disclosure will be partially provided in the following description, part of which will be apparent from the following description, or may be learned by practice of the present disclosure.

### BRIEF DESCRIPTION OF DRAWINGS

The above and/or additional aspects and advantages of the present disclosure will become apparent and comprehensible from the description of the examples in conjunction with the following drawings.
FIG. 1 shows a schematic diagram of the affinity between Hz140 and CD47-his determined by Fortebio;
FIG. 2 shows a schematic diagram of the binding activity of hz140 to U937 tumor cells analyzed by FACS;
FIG. 3 shows a schematic diagram of the binding activity of hz140 to CCRF tumor cells analyzed by FACS;
FIG. 4 shows a schematic diagram of the binding activity of hz140 to red blood cells analyzed by FACS;
FIG. 5 shows a schematic diagram of the activity of hz140 blocking SIRPa and CD47 on the surface of CCRF cells analyzed by FACS;
FIG. 6 shows a schematic diagram of the activity of hz140 blocking SIRPa and CD47 on the surface of U937 cells analyzed by FACS;
FIG. 7 shows a schematic diagram of the observation results of hz140 on the survival period of CCRF-SB leukemia model mice;
FIG. 8 shows a schematic diagram of the observation results of the *in vivo* pharmacodynamic of anti-CD47 antibody on human xenograft lymphoma Raji model;
FIG. 9 shows a schematic diagram of the quantitative results of signal intensity of the anti-tumor efficacy of 2MW1531 on immunodeficient mouse xenograft Raji hematologic tumor model via tail vein injection;
FIG. 10 shows a schematic diagram of the amino acid sequence alignment results of hz140 light chain and hz182 light chain;
FIG. 11 shows a schematic diagram of the analysis results of the binding activity of 2MW1531 to MC38-hPD-L1/hCD47 cells;
FIG. 12 shows a schematic diagram of the analysis results of the binding activity of 2MW1531 to A431 cells;
FIG. 13 shows a schematic diagram of the analysis results of the binding activity of 2MW1531 to human red blood cells;
FIG. 14 shows a schematic diagram of the results of the *in vitro* red blood cells agglutination test of the anti-human CD47/PD-L1 bispecific antibody;
FIG. 15 shows a schematic diagram of the experiment results of the anti-tumor efficacy of 2MW1531 on hPD-L1/hCD47/hSIRPα transgenic mice subcutaneously homografted MC38-hPDL1 mouse colon cancer tumor model (tumor volume);
FIG. 16 shows an *in vivo* imaging of the anti-tumor efficacy of 2MW1531 on the immunodeficient mouse xenograft Raji hematologic tumor model via tail vein injection.

### DETAILED DESCRIPTION

Embodiments of the present disclosure are described in detail below, examples of which are shown in the drawings, wherein the same or similar reference numerals designate the same or similar elements or elements having the same or similar functions throughout. The examples described below by referring to the drawings are exemplary, which are only intended to illustrate the present disclosure and should not be construed as limiting the present disclosure.

It should be noted that the terms "the first" and "the second" are only used for descriptive purposes, and should not be construed as indicating or implying relative importance or implicitly indicating the number of indicated technical features. Therefore, a feature defined as "the first" and "the second" may explicitly or implicitly include one or more of the features. Further, in the description of the present disclosure, unless otherwise specified, "multiple" means two or more than two.

The solutions of the present disclosure will be illustrated below in conjunction with examples. Those skilled in the art will understand that the following examples are only for illustrating the present disclosure and should not be construed as limiting the scope of the present disclosure. Examples without specific techniques or conditions indicated are carried out according to the techniques or conditions described in the literature in the art (for example, reference may be made to "Molecular Cloning: A Laboratory Manual" written by J. Sambrook et al., translated by Huang Peitang et al., third edition, China Science Publishing & Media) or according to the product instructions. The reagents or instruments used without manufacturers indicated are commercially available conventional products, which, for example, may be purchased from Sigma-Aldrich.

### Example 1: Production of control antibody and anti-CD47 antibody 140 with high affinity

CD47-mFc was used to immunize Balb/c mice for the preparation of hybridomas. Serum antibody titers were detected 34 days or 57 days after the initial immunization, respectively. Splenocytes and myeloma FO cells of the mice whose serum titer reached the requirements of the fusion experiment (serum antibody titer, 1:102400) were fused at an appropriate ratio (5:1) under the action of a fusion agent to prepare hybridomas monoclonal cells. The hybridoma cells were cultured using the selective medium R1640-HAT. Supernatant samples from 651 hybridoma were detected by ELISA on the 10th-14th day, including cell binding activity (FACS), CD47/SIPR blocking, and cross-reaction with recombinant cyno-CD47-mFc. Finally, clone 140 was screened out. The genes of the light chain and heavy chain variable regions of the above clone were cloned into a recombinant expression vector containing human Cκ and IgG4 gene reading frames. The vectors were transiently expressed in HEK293 cells, and the expressed products were purified to obtain pure protein products for analysis. Amino acid sequences of the light chain and heavy chain variable regions of the murine antibody clone 140 were set forth in SEQ ID NO: 43 and SEQ ID NO: 44, respectively.
SEQ ID NO: 43, Amino acid sequence of light chain variable region of murine antibody 140 
DIQMTQTTSSLSASLGDRVTISCRASQDITNYLNWYQQKPDGTVKLLIYYTSRL
HSGVPSRFSGSGSGTDYSLTITNLEQEDIATYFCQQGDTFPRTFGGGTKLEIKR
SEQ ID NO: 44, Amino acid sequence of heavy chain variable region of murine antibody 140

EVQLQQSGPELIKPGPSVKMSCKASGYKFTNYVMHWVKQKPGQGLEWIGYINP
YNDGIKYNEKFTGKATLTSDKSSNTAYMELISLTSEDSAVYYCAREGDFYANYGRLGFAY
WGQGTLVTVSA

The following antibodies were prepared as reference antibodies, all in the form of IgG4. The recombinant expression of the control antibody was realized by conventional molecular biology methods, and the expression and purification of the control antibody were completed by transient expression in HEK293. The antibody hu5F9 (US2015/0183874A1, hu5F9V2, with sequences of the light chain and heavy chain variable regions set forth in SEQ ID NO: 35 and SEQ ID NO: 36, respectively) researched by Forty Seven Company clinically, and the anti-CD47 antibody 1F8 (CN 110582515 A, with light chain and heavy chain variable regions set forth in SEQ ID NO: 37 and SEQ ID NO: 38, respectively) of I-Mab Company were prepared.
SEQ ID NO: 35, Amino acid sequence of light chain variable region of hu5F9 
DIVMTQSPLSLPVTPGEPASISCRSSQSIVYSNNGNTYLGWYLQKPGQSPQLLIY
KVSNRFSGVPDRFSGSGSGTDFTLKISRVEAEDVGVYYCFQGSHVPYTFGQGTKLEIKR
SEQ ID NO: 36, Amino acid sequence of heavy chain variable region of hu5F9 
QVQLVQSGAEVKKPGASVKVSCKASGYTFTNYNMHWVRQAPGQRLEWMGTI
YPGNDDTSYNQKFKDRVTITADTSASTAYMELSSLRSEDTAVYYCARGGYRAMDYWGQ
 GTLVTVSS
SEQ ID NO: 37, Amino acid sequence of light chain variable region of 1F8 
DIVMTQSPDSLAVSLGERATINCKSSQSVLYSSNNRNYLAWYQQKPGQPPKLLIN
QASTRASGVPDRFSGSGSGTEFTLIISSLHAEDVAIYYCQQYYTPPLAFGGGTKLEIKR
SEQ ID NO: 38, Amino acid sequence of heavy chain variable region of 1F8

KVQLVESGGGLVKPGGSLRLSCAASGFFTFSNAWMNWVRQAPGKGLEWVGRI
KRKTDGETTDYAAPVKGRFSISRDDSKNTLYLQMNSLKTEDTAVYYCAGSNRAFDIWGQ
GTMVTVSA

### Example 2: Humanization modification of anti-human CD47 antibody 140 and evaluation

### 2.1 Humanization of murine antibody 140

Murine antibody 140 was humanized using IGKV1-33^{∗}01|IGKJ4^{∗}02 and IGHV1-3^{∗}01|IGHJ1^{∗}01 as germline templates. The humanized light chain and heavy chain had sequences set forth in SEQ ID NO: 8 and SEQ ID NO: 5.
SEQ ID NO: 8, Amino acid sequence of heavy chain variable region of humanized 140 
QVQLVQSGAEVKKPGASVKVSCKASGYKFTNYVMHWVRQAPGQRLEWMGYI
NPYNDGIKYNEKFTGRVTITRDTSASTAYMELSSLRSEDTAVYYCAREGDFYANYGRLGF
AYWGQGTLVTVSS
SEQ ID NO: 5, Amino acid sequence of light chain variable region of humanized 140

DIQMTQSPSSLSASVGDRVTITCRASQDITNYLNWYQQKPGKAPKLLIYYTSRL
HSGVPSRFSGSGSGTDYTFTISSLQPEDIATYFCQQGDTFPRTFGGGTKVEIKR

### 2.2 Evaluation of binding activity and blocking activity on cell surface

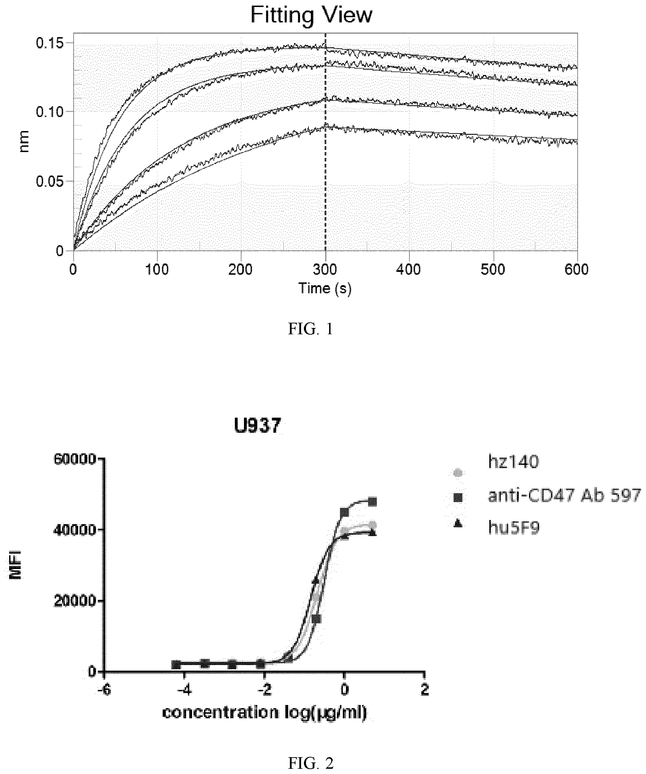

Humanized 140 was evaluated using Fortebio and FACS, respectively. Fortebio results are shown in FIG. 1 and Table 1. Different concentrations of humanized 140 (hz140) were loaded onto the surface immobilized with CD47-His, where the concentrations of hz140 were 75 nm, 60 nm, 30 nm, and 15 nm respectively. It was determined that kon(1/Ms)=2.47E+05, kdis(1/s)=3.70E-04, and KD (M)=1.50E-09, demonstrating that the humanized hz140 maintained a good affinity.

**Table 1: Affinity constants of Hz140 binding to CD47-his determined by Fortebio**

| Sample ID | Loading Sample ID | Cone. (nM) | Response | KD (M) | kon(1/Ms) | kdis(1/s) |
|---|---|---|---|---|---|---|
| CD47-His | Hz140 | 75 | 0.1462 | 1.50E-09 | 2.47E+05 | 3.70E-04 |
| CD47-His | Hz140 | 60 | 0.1326 | 1.50E-09 | 2.47E+05 | 3.70E-04 |
| CD47-His | Hz140 | 30 | 0.1069 | 1.50E-09 | 2.47E+05 | 3.70E-04 |
| CD47-His | Hz140 | 15 | 0.0863 | 1.50E-09 | 2.47E+05 | 3.70E-04 |

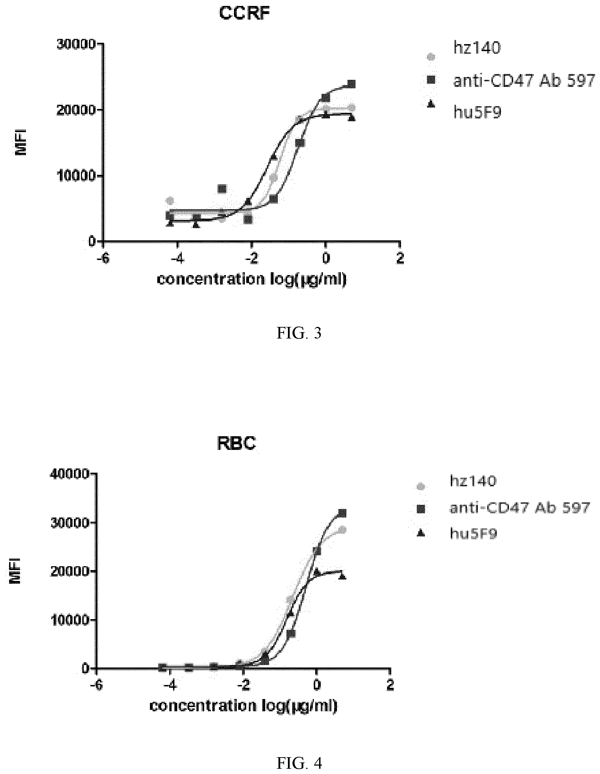

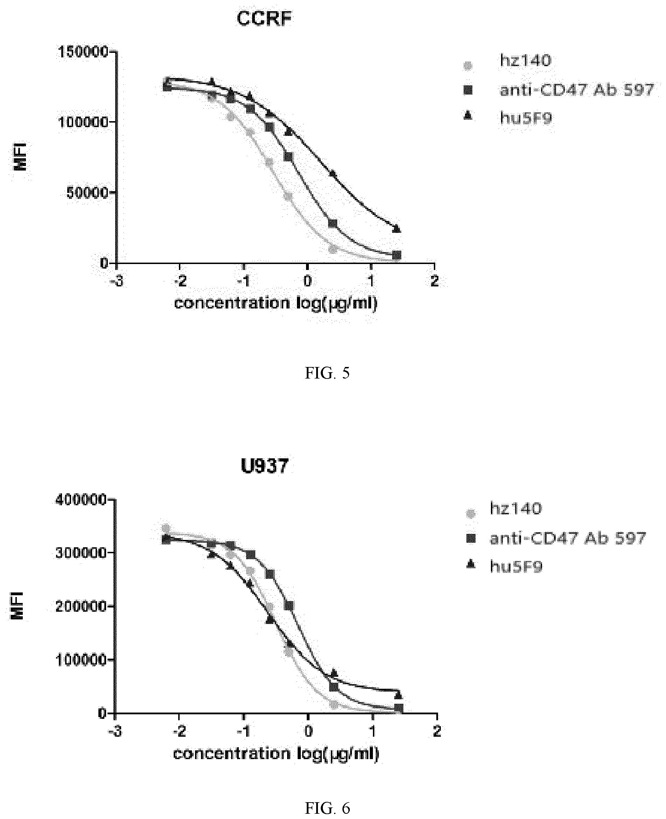

The activity of hz140 binding to CD47 antigen on the surface of U937, CCRF and human RBC cells was evaluated by FACS. The results are shown in FIG. 2-FIG. 4. The binding curves of hz140 and the control antibody hu5F9 on the three types of cells were similar, indicating that the binding activity of hz140 on the surface of the three types of cells was basically equivalent to that of the control antibody hu5F9. The results of binding and blocking experiments between SIRPa-Fc (AAH26692.1, FITC labeled rhSIRPa-Fc) and CD47 on the surface of U937 and CCRF cells are shown in FIG. 5 and FIG. 6. On U937 cells with high CD47 abundance, the blocking effect of hz140 was basically equivalent to that of hu5F9; for CCRF cells with low CD47 abundance, the blocking activity of hz140 was significantly stronger than that of hu5F9.

### 2.3 Antitumor effect of Hz140 on human acute B lymphocyte leukemia model CCRF-SB

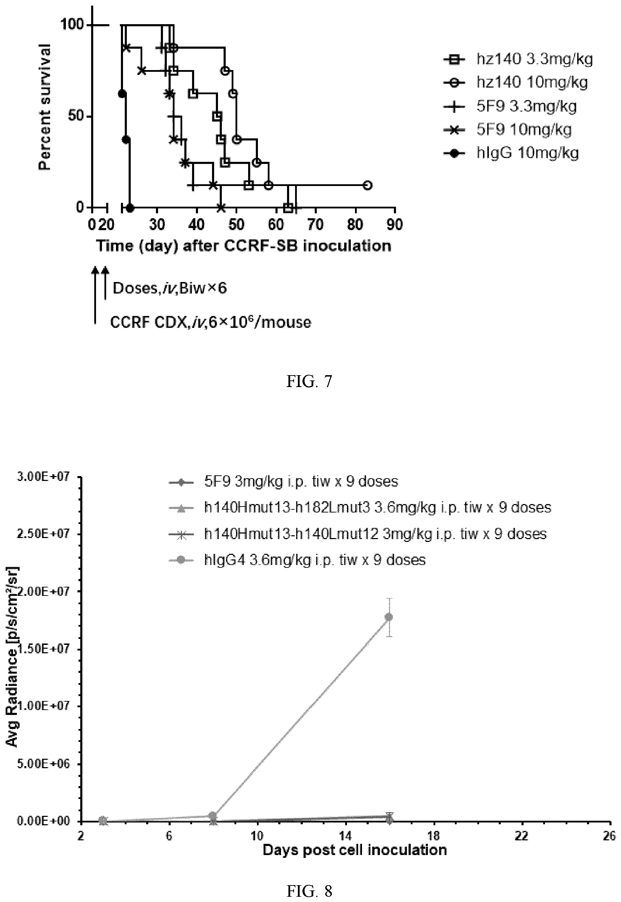

Balb/c nude mice, 6-8 weeks old, were intraperitoneally injected with cyclophosphamide at 2 mg/mouse/200 µl for immunosuppression. After 48 hours, the mice were inoculated via tail vein with 6.25×10⁶ freshly treated CCRF-SB cells. Five days after inoculation (D5), the mice were weighed and randomly assigned to each experimental group according to the body weight data, with 8 mice in each group. On the day of grouping, the mice were administered with different amounts of the test antibodies (i.v., twice a week, 6 times in total), and the survival period of the mice was observed. The results are shown in FIG. 7. Compared with the negative control hIgG, the positive control antibody hu5F9 can prolong the survival period of the model mice. However, there was no significant difference between the high-dose group (10 mg/kg) and the low-dose group (3.3 mg/kg) of hu5F9. Hz140 not only significantly prolonged the survival period of the model mice compared with the negative control, but also showed better effect than the positive control antibody hu5F9 at the same dose. In addition, the high-dose group of hz140 had better effect than the low-dose group of hz140, suggesting that hz140 prolonged the survival period of the model mice in a dose-dependent manner.

The above results show that the hz140 provided by the present disclosure had a better therapeutic effect than hu5F9 when administered at the same dose in the human acute B lymphoblastic leukemia animal model, and exhibited dose-dependency and higher reliability.

### Example 3: Affinity modification of hz140

On the basis of the light chain and heavy chain variable region sequence (SEQ ID NO: 8) of the humanized hz140 antibody, the heavy chain CDRs sequences were mutated. The Koff value of the antibody binding to CD47 was reduced to reduce the impact of the antibody on RBC and PLT. Multiple heavy chain mutants of humanized hz140 were constructed. The heavy chain of humanized hz140 was subjected to amino acid site-directed mutations. The mutant heavy chain was paired with the light chain of humanized hz140, and the affinity was determined. The comparison results are shown in Table 2. Further mutations were performed on the basis of hm10 to obtain a final sequence of 140Hm13 set forth in SEQ ID NO: 23.

**Table 2. Sequence information of the heavy chain mutants of hz140-Hm/hz140L and affinity data thereof when using recombinant CD47 extracellular region as antigen**

| Number | CDR1 | CDR2 | CDR3 | KD(M) | kon(1/Ms ) | kdis(1/s) |
|---|---|---|---|---|---|---|
| 140H | NYVMH (SEQ ID NO: 76) | YINPYNDGIKYNEKF TG (SEQ ID NO: 79) | EGDFYANYGRLGFAY (SEQ ID NO: 85) | 6.88E-10 | 3.20E+05 | 2.20E-04 |
| 140Hm1 | **S**YVMH (SEQ ID NO: 77) | YINPYNDGIKYNEKF TG (SEQ ID NO: 79) | EGDFYANYGRLGFAY (SEQ ID NO: 85) | 3.65E-09 | 2.96E+05 | 1.08E-03 |
| 140Hm2 | NY**A**MH (SEQ ID NO: 87) | YINPYNDGIKYNEKF TG (SEQ ID NO: 79) | EGDFYANYGRLGFAY (SEQ ID NO: 85) | 2.67E-09 | 2.66E+05 | 7.10E-04 |
| 140Hm3 | NYVM**S** (SEQ ID NO: 78) | YINPYNDGIKYNEKF TG (SEQ ID NO: 79) | EGDFYANYGRLGFAY (SEQ ID NO: 85) | 5.12E-09 | 2.01E+05 | 1.03E-03 |
| 140Hm4 | NYVMH (SEQ ID NO: 76) | W INPYNDGIKYNEKF TG (SEQ ID NO: 80) | EGDFYANYGRLGFAY (SEQ ID NO: 85) | 6.17E-09 | 3.50E+05 | 2.16E-03 |
| 140Hm5 | NYVMH (SEQ ID NO: 76) | YINPGNDGIKYNEKF TG (SEQ ID NO: 81) | EGDFYANYGRLGFAY (SEQ ID NO: 85) | 2.08E-08 | 2.24E+05 | 4.67E-03 |
| 140Hm6 | NYVMH (SEQ ID NO: 76) | YINPYNDGIKYNEKF QG (SEQ ID NO: 82) | EGDFYANYGRLGFAY (SEQ ID NO: 85) | 2.26E-08 | 3.13E+05 | 7.07E-03 |
| 140Hm7 | NYVMH (SEQ ID NO: 76) | YINPYNDGIKYNEKF TG (SEQ ID NO: 79) | EGDFYANYGRLGF**D**Y (SEQ ID NO: 86) | 6.14E-08 | 2.13E+05 | 1.31E-02 |
| 140Hm8 | NYVMH (SEQ ID NO: 76) | YINPYNDAIKYNEKF TG (SEQ ID NO: 83) | EGDFYANYGRLGFAY (SEQ ID NO: 85) | 3.87E-09 | 2.43E+05 | 9.40E-04 |
| 140Hm9 | NYVMH (SEQ ID NO: 76) | YINPYNEGIKYNEKF TG (SEQ ID NO: 84) | EGDFYANYGRLGFAY (SEQ ID NO: 85) | 3.92E-09 | 2.16E+05 | 8.47E-04 |
| 140Hm1 0 | NYVM**S** (SEQ ID NO: 78) | YINPYNDAIKYNEKF TG (SEQ ID NO: 83) | EGDFYANYGRLGFAY (SEQ ID NO: 85) | 7.83E-09 | 2.58E+05 | 2.02E-03 |

SEQ ID NO: 23, Heavy chain mutant 140Hm13 of humanized 140 antibody

EVQLVQSGAEVKKPGASVKVSCKASGYKFTNYVMSWVRQAPGQRLEWMGYI
NPYNDAIKYNEKFTGRVTITRDTSASTAYMELSSLRSEDTAVYYCAREGDFYANYGRLGF
AYWGQGTLVTVSS

### Example 4: Screening of mutants with reduced binding activity to red blood cells

140Hm13 was used as a heavy chain, and subjected to mutant design and combination with h140L (SEQ ID NO: 5) and a similar light chain sequence hz182L (SEQ ID NO: 1) of another heterologous antibody. The difference between the binding of the mutant to CD47 on the surface of tumor cells and CD47 on the surface of red blood cells was evaluated. The sequence of the light chain variable region of hz140 was subjected to mutation design, and the mutant design is shown in Table 3 below. The light chain of hz182 (h182L) was subjected to mutation design, and the mutant design is shown in Table 4 below. The binding of different mutants to tumor cells (1 µg/mL) and red blood cells (150 µg/mL) was evaluated by FACS to find mutation sites with binding differences.

SEQ ID NO: 1, Sequence of light chain hz182L of humanized 182 antibody

DIQMTQSPSSLSASVGDRVTITCRASQDISNYLNWYQQKPGKAPKLLLYYTSRL
HSGVPSRFSGSGSGTDYTLTISSLQPEDFATYFCQQGAGRPYTFGGGTKVEIK

The heavy chain mutant h140Hm13 of hz140 was combined and co-expressed with a series of light chain mutants of h140 (h140Lm1∼h140Lm18) and light chain mutants of h182 (hz182-Lm1∼hz182-Lm11) respectively to prepare recombinant antibodies, and a series of anti-CD47-specific antibodies that did not specifically bind to red blood cells were identified. According to the results of the mutants of h140L and h182L in Table 3 and Table 4, it was found that the ability of h140Lmut8-mut10 binding to red blood cells was significantly reduced. In addition, the activity of h140Lmut8-mut10 binding to tumor was maintained at a certain degree even though the binding activity was reduced. In particular, mut8 and mut10 completely lost the ability of binding to red blood cells, yet still maintained the ability of binding to tumor cells, suggesting that the mutation of the 96th amino acid located in the CDR3 region from R to Y was a key for binding to red blood cell. The reverse mutation of h182L also demonstrated that the mutation of h182L into h182Lmut8 (the mutation of Y into R) restored the activity of binding to red blood cells.

**Table 3. Amino acid sequences of mutants of h140L**

| Number | FR1 | LCDR1 | FR2 | LCDR2 | FR3 | LCDR3 | FACS (MFI) | |
|---|---|---|---|---|---|---|---|---|
| | | | | | | | Tumor cell 1nM | hRBC 500nM |
| h140L | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTFTISSLQPEDIAT YFC (SEQ ID NO: 53) | QQGDTFPRT (SEQ ID NO: 56) | 102341 | 27423 |
| hl40Lml | DIQMTQSPSSL SASVGDRVTIT C (SEQ ID NO 45) | RASQDISNYLN (SEQ ID NO: 47) | WYQQKPGKAPKL LIY (SEQ ID NO: 48) | YTSRLHS (SEQ ID NO: 50) | GVPSRFSGSGSGTD YTFTISSLQPEDIAT YFC (SEQ ID NO: 53) | QQGDTFPRT (SEQ ID NO: 56) | / | 28673 |
| h140Lm2 | DIQMTQSPSSL SASVGDRVTIT C (SEQ ID NO 45) | RASQDITNYLN (SEQ ID NO: 46) | WYQQKPGKAPKL LLY (SEQ ID NO: 49) | YTSRLHS (SEQ ID NO: 50) | GVPSRFSGSGSGTD YTFTISSLQPEDIAT YFC (SEQ ID NO: 53) | QQGDTFPRT (SEQ ID NO: 56) | / | 26084 |
| h140Lm3 | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTLTISSLQPEDIAT YFC (SEQ ID NO: 54) | QQGDTFPRT (SEQ ID NO: 56) | / | 30593 |
| h140Lm4 | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTFTISSLQPEDFAT YFC (SEQ ID NO: 55) | QQGDTFPRT (SEQ ID NO: 56) | / | 24157 |
| h140Lm5 | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTFTISSLQPEDIAT YFC (SEQ ID NO: 53) | QQGATFPRT (SEQ ID NO: 57) | / | 27296 |
| h140Lm6 | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTFTISSLQPEDIAT YFC (SEQ ID NO: 53) | QQGDGFPRT (SEQ ID NO: 58) | / | 27194 |
| h140Lm7 | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTFTISSLQPEDIAT YFC (SEQ ID NO: 53) | QQGDTRPRT (SEQ ID NO: 59) | 40234 | 16715 |
| hl40Lm8 | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTFTISSLQPEDIAT YFC (SEQ ID NO: 53) | QQGDTRPYT (SEQ ID NO: 60) | 26397 | 1759.6 |
| h140Lm9 | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTFTISSLQPEDIAT YFC (SEQ ID NO: 53) | QQGAGRPRT (SEQ ID NO: 61) | 34599 | 17736 |
| h140Lm10 | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTFTISSLQPEDIAT YFC (SEQ ID NO: 53) | QQGAGRPYT (SEQ ID NO: 62) | 40857 | 936.1 |
| hl40Lmll | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLSS (SEQ ID NO: 51) | GVPSRFSGSGSGTD YTFTISSLQPEDIAT YFC (SEQ ID NO: 53) | QQGDTRPYT (SEQ ID NO: 60) | 19362 | 788.6 |
| h140Lm12 | DIQMTQSPSSL SASVGDRVTIT C (SEQ ID NO 45) | RASQDITNYLN (SEQ ID NO: 46) | WYQQKPGKAPKL LIY (SEQ ID NO: 48) | YTSRLQS (SEQ ID NO: 52) | GVPSRFSGSGSGTD YTFTISSLQPEDIAT YFC (SEQ ID NO: 53) | QQGDTRPYT (SEQ ID NO: 60) | 26363 | 945.6 |
| h140Lm13 | DIQMTQSPSSL SASVGDRVTIT C (SEQ ID NO: 45) | RASQDITNYLN (SEQ ID NO: 46) | WYQQKPGKAPKL LIY (SEQ ID NO: 48) | YTSRLHS (SEQ ID NO: 50) | GVPSRFSGSGSGTD YTFTISSLQPEDIAT YFC (SEQ ID NO: 53) | QQGDTFPYT (SEQ ID NO: 63) | 10277 | 1759.6 |
| h140Lm14 | DIQMTQSPSSL SASVGDRVTIT C (SEQ ID NO: 45) | RASQDITNYLN (SEQ ID NO: 46) | WYQQKPGKAPKL LIY (SEQ ID NO: 48) | YTSRLQS (SEQ ID NO: 52) | GVPSRFSGSGSGTD YTFTISSLQPEDIAT YFC (SEQ ID NO: 53) | QQGDTYPYT (SEQ ID NO: 64) | 7521.6 | 921.2 |
| h140Lm15 | DIQMTQSPSSL SASVGDRVTIT C (SEQ ID NO: 45) | RASQDITNYLN (SEQ ID NO: 46) | WYQQKPGKAPKL LIY (SEQ ID NO: 48) | YTSRLQS (SEQ ID NO: 52) | GVPSRFSGSGSGTD YTFTISSLQPEDIAT YFC (SEQ ID NO: 53) | QQGDTKPYT (SEQ ID NO: 65) | 15689 | 953.5 |
| h140Lm16 | DIQMTQSPSSL SASVGDRVTIT C (SEQ ID NO: 45) | RASQDITNYLN (SEQ ID NO: 46) | WYQQKPGKAPKL LIY (SEQ ID NO: 48) | YTSRLQS (SEQ ID NO: 52) | GVPSRFSGSGSGTD YTFTISSLQPEDIAT YFC (SEQ ID NO: 53) | QQGDTSPYT (SEQ ID NO: 66) | 12223.8 | 721.4 |
| h140Lm17 | DIQMTQSPSSL SASVGDRVTIT C (SEQ ID NO: 45) | RASQDITNYLN (SEQ ID NO: 46) | WYQQKPGKAPKL LIY (SEQ ID NO: 48) | YTSRLQS (SEQ ID NO: 52) | GVPSRFSGSGSGTD YTFTISSLQPEDIAT YFC (SEQ ID NO: 53) | QQGDTEPYT (SEQ ID NO: 67) | 11161.8 | 689.4 |
| h140Lm18 | DIQMTQSPSSL SASVGDRVTIT C (SEQ ID NO: 45) | RASQDITNYLN (SEQ ID NO: 46) | WYQQKPGKAPKL LIY (SEQ ID NO: 48) | YTSRLQS (SEQ ID NO: 52) | GVPSRFSGSGSGTD YTFTISSLQPEDIAT YFC (SEQ ID NO: 53) | QQGDTNPYT (SEQ ID NO: 68) | 9922 | 697 |

**Table 4. Amino acid sequences of h182L mutants**

| Number | CDR1 | FR2 | FR3 | CDR3 | FACS (MFI) | |
|---|---|---|---|---|---|---|
| | | | | | Tumor cell (2nM) | RBC (200nM ) |
| hz182-L | RASQDISNYL N (SEQ ID NO: 47) | WYQQKPGKAPKLLL Y (SEQ ID NO: 49) | GVPSRFSGSGSGTDYTLTI SSLQPEDFATYFC (SEQ ID NO: 69) | QQGAGRPYT (SEQ ID NO: 62) | 25826.8 | 2059.3 |
| hzl82-Lml | RASQDISNYL N (SEQ ID NO: 47) | WYQQKPGKAPKLLI Y (SEQ ID NO: 48) | GVPSRFSGSGSGTDYTLTI SSLQPEDFATYFC (SEQ ID NO: 69) | QQGAGRPYT (SEQ ID NO: 62) | 15495.2 | 2324.5 |
| hzl82-Lm2 | RASQDISNYL N (SEQ ID NO: 47) | WYQQKPGKAPKLLL Y (SEQ ID NO: 49) | GVPSRFSGSGSGTDYTFTI SSLQPEDFATYFC (SEQ ID NO: 55) | QQGAGRPYT (SEQ ID NO: 62) | 30434.9 | 1668.2 |
| hz182-Lm3 | RASQDISNYL N (SEQ ID NO: 47) | WYQQKPGKAPKLLL Y (SEQ ID NO: 49) | GVPSRFSGSGSGTDYTLTI SSLQPEDIATYFC (SEQ ID NO: 54) | QQGAGRPYT (SEQ ID NO: 62) | 30043.8 | 874.4 |
| hz182-Lm4 | RASQDITNYL N (SEQ ID NO: 46) | WYQQKPGKAPKLLL Y (SEQ ID NO: 49) | GVPSRFSGSGSGTDYTLTI SSLQPEDFATYFC (SEQ ID NO: 69) | QQGAGRPYT (SEQ ID NO: 62) | 28502.6 | 1843.1 |
| hz182-Lm5 | RASQDISNYL N (SEQ ID NO: 47) | WYQQKPGKAPKLLL Y (SEQ ID NO: 49) | GVPSRFSGSGSGTDYTLTI SSLQPEDFATYFC (SEQ ID NO: 69) | QQGDGRPYT (SEQ ID NO: 70) | 39933.2 | 3314.6 |
| hz182-Lm6 | RASQDISNYL N (SEQ ID NO: 47) | WYQQKPGKAPKLLL Y (SEQ ID NO: 49) | GVPSRFSGSGSGTDYTLTI SSLQPEDFATYFC (SEQ ID NO: 69) | QQGATRPYT (SEQ ID NO: 71) | 15919 | 694.7 |
| hz182-Lm7 | RASQDISNYL N (SEQ ID NO: 47) | WYQQKPGKAPKLLL Y (SEQ ID NO: 49) | GVPSRFSGSGSGTDYTLTI SSLQPEDFATYFC (SEQ ID NO: 69) | QQGAGFPYT (SEQ ID NO: 72) | 17786.5 | 348.7 |
| hzl82-Lm8 | RASQDISNYL N (SEQ ID NO: 47) | WYQQKPGKAPKLLL Y (SEQ ID NO: 49) | GVPSRFSGSGSGTDYTLTI SSLQPEDFATYFC (SEQ ID NO: 69) | QQGAGRPRT (SEQ ID NO: 61) | 39117.2 | 40790.8 |
| hz182-Lm9 | RASQDISNYL N (SEQ ID NO: 47) | WYQQKPGKAPKLLL Y (SEQ ID NO: 49) | GVPSRFSGSGSGTDYTLTI SSLQPEDFATYFC (SEQ ID NO: 69) | QQGDTFPYT (SEQ ID NO: 63) | 16365.3 | 1750.6 |
| hzl82-LmlO | RASQDISNYL N (SEQ ID NO: 47) | WYQQKPGKAPKLLL Y (SEQ ID NO: 49) | GVPSRFSGSGSGTDYTLTI SSLQPEDIATYFC (SEQ ID NO: 54) | QQGAGFPYT (SEQ ID NO: 72) | 18967.4 | 325.6 |
| hzl82-Lmll | RASQDISNYL N (SEQ ID NO: 47) | WYQQKPGKAPKLLL Y (SEQ ID NO: 49) | GVPSRFSGSGSGTDYTLTI SSLQPEDIATYFC (SEQ ID NO: 54) | QQGDTFPYT (SEQ ID NO: 63) | 20143.2 | 321.4 |

### Example 5: Preparation of initial monoclonal antibody

### 1. Preparation of anti-human PD-L1 monoclonal antibody

A humanized anti-human PD-L1 antibody was prepared according to a prior patent application (Patent Application CN201911419802.5). The humanized antibody comprised a light chain variable region (hz182-L) comprising an amino acid sequence set forth in SEQ ID NO: 1 and a coding nucleotide sequence set forth in SEQ ID NO: 2; and a heavy chain variable region (hz182-H) comprising an amino acid sequence set forth in SEQ ID NO: 3 and a coding nucleotide sequence set forth in SEQ ID NO: 4.

### 2. Preparation of anti-human CD47 monoclonal antibody

The anti-human CD47 antibody 140 prepared in Example 2 was used to prepare an anti-human CD47 monoclonal antibody, where the hz140 comprised a light chain variable region (hz140-L) comprising an amino acid sequence set forth in SEQ ID NO: 5 and a nucleotide sequence set forth in SEQ ID NO: 6; a light chain constant region comprising an amino acid sequence set forth in SEQ ID NO: 7; a heavy chain variable region (hz140-H) comprising an amino acid sequence set forth in SEQ ID NO: 8 and a nucleotide sequence set forth in SEQ ID NO: 9; and a heavy chain constant region comprising an amino acid sequence set forth in SEQ ID NO: 10.

### Example 6: Preparation of hybrid antibody of anti-CD47 antibody heavy chain/anti-PD-L1 antibody light chain (hz140-H/hz182-L)

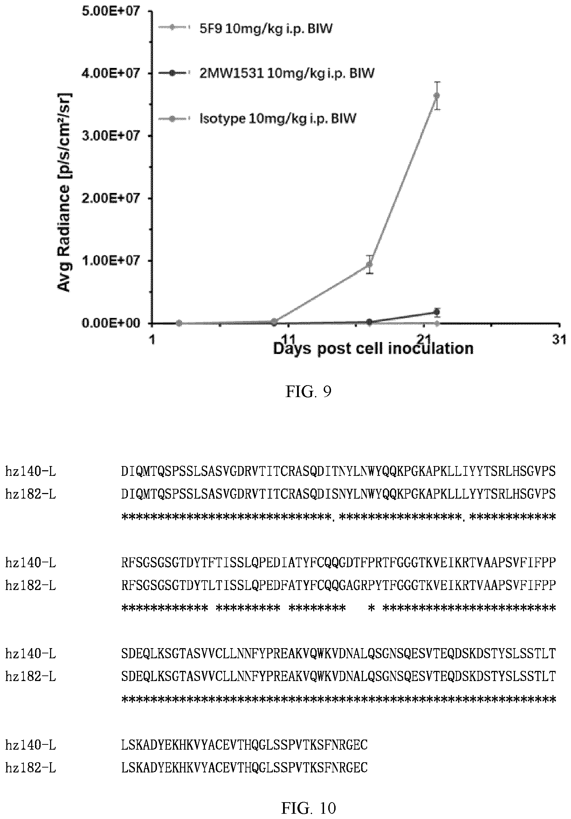

By sequence alignment, it was found that the light chain of the hz182 antibody (hz182-L) was highly similar to the light chain of the hz140 antibody (hz140-L) in Example 2 (with 96.26% homology, see FIG. 10). The sequence alignment results in FIG. 10 show that there were a total of 8 amino acid residue differences in sequence structures of the light chain of hz140 and the light chain of hz182, where there was 1 amino acid residue difference between CDR1 regions, and 1 amino acid residue difference between FR2 regions, and there were 2 amino acid residue differences between FR3 regions, and 4 amino acid residue differences between CDR3 regions.

The light chain of hz182 (hz182-L) and the heavy chain of hz140 (hz140-H) were expressed in combination, and the expression supernatant was subjected to affinity purification to obtain an anti-human CD47 antibody hz140-H/hz182-L which had the same light chain with hz182. The affinity of the antibody was determined by Octet QKe instrument of Fortebio Company. The antibody was immobilized on the surface of a sensor with anti-human Fc capture antibody (ARC sensor), and reacted with a recombinant antigen of the extracellular region of CD47 (purchased from Sino Biological, Cat: 12283-H08H; with a working concentration of 100 nM) in the liquid phase. The kinetic parameters were determined. The results show that hz140-H-/hz182-L had good activity of binding to the CD47 recombinant protein (Table 5).

**Table 5. Results of affinity assay of hz140 and hz140-H/hz182-L**

| | KD (M) | kon(1/Ms) | kdis(1/s) |
|---|---|---|---|
| Hz140 | 1.89E-09 | 4.93E+05 | 9.33E-04 |
| hz140-H/hz182-L | 7.03E-09 | 1.54E+05 | 1.08E-03 |

### Example 7: Preparation of hybrid antibody of anti-CD47 antibody heavy chain mutant/anti-PD-L1 antibody light chain (hz140-Hm/hz182-L)

The heavy chain of the hybrid antibody hz140-H/hz182-L was genetically engineered, and the affinity of the recombinant antibody to CD47 was evaluated. Specifically, by analyzing the distribution frequency of amino acid types at each site in the variable region of the antibody, the amino acid sites that may affect the affinity of the antibody were subjected to rational mutation design, and mutations at different sites were combined. Then mutations were introduced into the corresponding sites of the coding gene of the antibody by site-directed mutation PCR to construct a mutant expression plasmid. Finally, the mutated heavy chain hz140-Hm and light chain hz182-L were expressed in combination, and the expression supernatant was subjected to affinity purification. The affinity between the obtained mutant antibody and the CD47 recombinant protein was determined by Fortebio. The antibody was immobilized on the surface of a sensor with the anti-human Fc capture antibody (ARC Sensor), and reacted with the recombinant antigen of the extracellular region of CD47 (with a working concentration of 100 nM) in the liquid phase. The duration for binding and dissociation was both 300 seconds. The kinetic parameters were determined. The mutation sites in the heavy chain hz140-H, and the affinity of the mutant hz140-Hm to recombinant CD47 are shown in Table 6.

### Example 8: Preparation of hybrid antibody of anti-CD47 antibody heavy chain mutant 13/anti-PD-L1 antibody light chain mutant (hz140-Hm13/hz182-Lm)

The light chain of the hybrid antibody hz140-Hm13/hz182-L was genetically engineered, and the affinity of the recombinant antibody to CD47 was evaluated. The light chain mutant was designed according to the following principle: the common amino acid residues between the light chain of the hz140 antibody and the light chain of the hz182 antibody were retained, and the differences of the amino acid residues were mutated, where the mutated amino acid residue was preferably the amino acid residues at this site in the light chain of the hz140 antibody or in the light chain of the hz182 antibody, or another amino acids. Subsequently, the affinity between these mutants and CD47 was determined by the method in Example 2. The mutation sites in the light chain of hz182-L, and the affinity of the mutant hz182-Lm to recombinant CD47 are shown in Table 7.

**Table 6. Sequence information of heavy chain mutants of hz140-Hm/hz182-L and affinity data when using recombinant CD47 extracellular region as antigen**

| Sequen ce number | Number | FR1 | CDR1 | CDR2 | CDR3 | KD (M) | kon(1/Ms) | kdis(1/s) |
|---|---|---|---|---|---|---|---|---|
| 8 | hz140-H | QVQLVQSGAEVKKP GASVKVSCKASGYK FT (SEQ ID NO: 73) | NYVMH (SEQ ID NO: 76) | YINPYNDGIKY NEKFTG (SEQ ID NO: 79) | EGDFYANYG RLGFAY (SEQ ID NO: 85) | 7.19E-10 | 3.15E+05 | 2.26E-04 |
| 11 | hz140-Hm 1 | QVQLVQSGAEVKKP GASVKVSCKASGYT FT (SEQ ID NO: 74) | NYVMH (SEQ ID NO: 76) | YINPYNDGIKY NEKFTG (SEQ ID NO: 79) | EGDFYANYG RLGFAY (SEQ ID NO: 85) | 1.67E-09 | 3.25E+05 | 5.42E-04 |
| 12 | hz140-Hm 2 | QVQLVQSGAEVKKP GASVKVSCKASGYK FT (SEQ ID NO: 73) | SYVMH (SEQ ID NO: 77) | YINPYNDGIKY NEKFTG (SEQ ID NO: 79) | EGDFYANYG RLGFAY (SEQ ID NO: 85) | 1.71E-09 | 3.17E+05 | 5.41E-04 |
| 13 | hz140-Hm 3 | QVQLVQSGAEVKKP GASVKVSCKASGYK FT (SEQ ID NO: 73) | NYAMH (SEQ ID NO: 87) | YINPYNDGIKY NEKFTG (SEQ ID NO: 79) | EGDFYANYG RLGFAY (SEQ ID NO: 85) | 1.03E-08 | 2.99E+05 | 3.08E-03 |
| 14 | hz140-Hm 4 | QVQLVQSGAEVKKP GASVKVSCKASGYK FT (SEQ ID NO: 73) | NYVMS (SEQ ID NO: 78) | YINPYNDGIKY NEKFTG (SEQ ID NO: 79) | EGDFYANYG RLGFAY (SEQ ID NO: 85) | 5.97E-09 | 2.86E+05 | 1.71E-03 |
| 15 | hz140-Hm 5 | QVQLVQSGAEVKKP GASVKVSCKASGYK FT (SEQ ID NO: 73) | NYVMH (SEQ ID NO: 76) | WINPYNDGIKY NEKFTG (SEQ ID NO: 80) | EGDFYANYG RLGFAY (SEQ ID NO: 85) | 6.82E-09 | 1.51E+05 | 1.03E-03 |
| 16 | hz140-Hm 6 | QVQLVQSGAEVKKP GASVKVSCKASGYK FT (SEQ ID NO: 73) | NYVMH (SEQ ID NO: 76) | YINPGNDGIKY NEKFTG (SEQ ID NO: 81) | EGDFYANYG RLGFAY (SEQ ID NO: 85) | 8.55E-09 | 3.70E+05 | 3.16E-03 |
| 17 | hz140-Hm 7 | QVQLVQSGAEVKKP GASVKVSCKASGYK FT (SEQ ID NO: 73) | NYVMH (SEQ ID NO: 76) | YINPYNDGIKY NEKFQG (SEQ ID NO: 82) | EGDFYANYG RLGFAY (SEQ ID NO: 85) | 6.18E-08 | 1.24E+05 | 7.67E-03 |
| 18 | hz140-Hm 8 | QVQLVQSGAEVKKP GASVKVSCKASGYK FT (SEQ ID NO: 73) | NYVMH (SEQ ID NO: 76) | YINPYNDGIKY NEKFTG (SEQ ID NO: 79) | EGDFYANYG RLGFDY (SEQ ID NO: 86) | 4.25E-08 | 2.13E+05 | 9.07E-03 |
| 19 | hz140-Hm 9 | QVQLVQSGAEVKKP GASVKVSCKASGYK FT (SEQ ID NO: 73) | NYVMH (SEQ ID NO: 76) | YINPYNDAIKY NEKFTG (SEQ ID NO: 83) | EGDFYANYG RLGFAY (SEQ ID NO: 85) | <1.0E-1 2 | 2.23E+05 | <1.0E-07 |
| 20 | hz140-Hm 10 | QVQLVQSGAEVKKP GASVKVSCKASGYK FT (SEQ ID NO: 73) | NYVMH (SEQ ID NO: 76) | YINPYNEGIKY NEKFTG (SEQ ID NO: 84) | EGDFYANYG RLGFAY (SEQ ID NO: 85) | <1.0E-1 2 | 2.06E+05 | <1.0E-07 |
| 21 | hz140-Hm 11 | QVQLVQSGAEVKKP GASVKVSCKASGYK FT (SEQ ID NO: 73) | NYVMS (SEQ ID NO: 78) | YINPYNDAIKY NEKFTG (SEQ ID NO: 83) | EGDFYANYG RLGFAY (SEQ ID NO: 85) | 6.53E-09 | 2.60E+05 | 1.70E-03 |
| 22 | hz140-Hm 12 | QVQLVQSGAEVKKP GASVKVSCKASGYT FT (SEQ ID NO: 74) | NYVMS (SEQ ID NO: 78) | YINPYNDAIKY NEKFTG (SEQ ID NO: 83) | EGDFYANYG RLGFAY (SEQ ID NO: 85) | 1.47E-08 | 2.24E+05 | 3.28E-03 |
| 23 | hz140-Hm 13 | EVQLVQSGAEVKKP GASVKVSCKASGYK FT (SEQ ID NO: 75) | NYVMS (SEQ ID NO: 78) | YINPYNDAIKY NEKFTG (SEQ ID NO: 83) | EGDFYANYG RLGFAY (SEQ ID NO: 85) | 6.20E-09 | 2.63E+05 | 1.63E-03 |

**Table 7. Sequence information of light chain mutants of hz140-Hm13/hz182-L and affinity thereof to recombinant CD47**

| Seque nce numbe r | Number | CDR1 | FR2 | FR3 | CDR3 | KD (M) | kon(1/Ms) |
|---|---|---|---|---|---|---|---|
| 1 | hz182-L | RASQDISNY LN (SEQ ID NO: 47) | WYQQKPGK APKLLLY (SEQ ID NO: 49) | GVPSRFSGSGSGTDY TLTISSLQPEDFATYF C (SEQ ID NO: 69) | QQGAGRPYT (SEQ ID NO: 62) | 5.97E-09 | 1.25E+05 |
| 24 | hzl82-Lm1 | RASQDISNY LN (SEQ ID NO: 47) | WYQQKPGK APKLLIY (SEQ ID NO: 48) | GVPSRFSGSGSGTDY TLTISSLQPEDFATYF C (SEQ ID NO: 69) | QQGAGRPYT (SEQ ID NO: 62) | 7.06E-09 | 1.34E+05 |
| 25 | hz182-Lm2 | RASQDISNY LN (SEQ ID NO: 47) | WYQQKPGK APKLLLY (SEQ ID NO: 49) | GVPSRFSGSGSGTDY TFTISSLQPEDFATYF C (SEQ ID NO: 55) | QQGAGRPYT (SEQ ID NO: 62) | 1.41E-06 | 9.47E+04 |
| 26 | hz182-Lm3 | RASQDISNY LN (SEQ ID NO: 47) | WYQQKPGK APKLLLY (SEQ ID NO: 49) | GVPSRFSGSGSGTDY TLTISSLQPEDIATYF C (SEQ ID NO: 54) | QQGAGRPYT (SEQ ID NO: 62) | 1.67E-08 | 1.45E+05 |
| 27 | hz182-Lm4 | RASQDITNY LN (SEQ ID NO: 46) | WYQQKPGK APKLLLY (SEQ ID NO: 49) | GVPSRFSGSGSGTDY TLTISSLQPEDFATYF C (SEQ ID NO: 69) | QQGAGRPYT (SEQ ID NO: 62) | 9.89E-07 | 8.79E+04 |
| 28 | hz182-Lm5 | RASQDISNY LN (SEQ ID NO: 47) | WYQQKPGK APKLLLY (SEQ ID NO: 49) | GVPSRFSGSGSGTDY TLTISSLQPEDFATYF C (SEQ ID NO: 69) | QQGDGRPYT (SEQ ID NO: 70) | 6.67E-07 | 7.99E+04 |
| 29 | hz182-Lm6 | RASQDISNY LN (SEQ ID NO: 47) | WYQQKPGK APKLLLY (SEQ ID NO: 49) | GVPSRFSGSGSGTDY TLTISSLQPEDFATYF C (SEQ ID NO: 69) | QQGATRPYT (SEQ ID NO: 71) | 1.25E-08 | 1.14E+05 |
| 30 | hz182-Lm7 | RASQDISNY LN (SEQ ID NO: 47) | WYQQKPGK APKLLLY (SEQ ID NO: 49) | GVPSRFSGSGSGTDY TLTISSLQPEDFATYF C (SEQ ID NO: 69) | QQGAGFPYT (SEQ ID NO: 72) | 1.66E-08 | 2.01E+05 |
| 31 | hz182-Lm8 | RASQDISNY LN (SEQ ID NO: 47) | WYQQKPGK APKLLLY (SEQ ID NO: 49) | GVPSRFSGSGSGTDY TLTISSLQPEDFATYF C (SEQ ID NO: 69) | QQGAGRPRT (SEQ ID NO: 61) | 1.29E-08 | 2.34E+05 |
| 32 | hz182-Lm9 | RASQDISNY LN (SEQ ID NO: 47) | WYQQKPGK APKLLLY (SEQ ID NO: 49) | GVPSRFSGSGSGTDY TLTISSLQPEDFATYF C (SEQ ID NO: 69) | QQGDTFPYT (SEQ ID NO: 63) | 3.89E-08 | 1.32E+05 |
| 33 | hz182-Lm10 | RASQDISNY LN (SEQ ID NO: 47) | WYQQKPGK APKLLLY (SEQ ID NO: 49) | GVPSRFSGSGSGTDY TLTISSLQPEDIATYF C (SEQ ID NO: 54) | QQGAGFPYT (SEQ ID NO: 72) | 5.14E-09 | 1.76E+05 |
| 34 | hz182-Lm11 | RASQDISNY LN (SEQ ID NO: 47) | WYQQKPGK APKLLLY (SEQ ID NO: 49) | GVPSRFSGSGSGTDY TLTISSLQPEDIATYF C (SEQ ID NO: 54) | QQGDTFPYT (SEQ ID NO: 63) | 5.31E-08 | 1.03E+05 |

### Example 9: Preparation and effect evaluation of anti-CD47/PD-L1 bispecific antibody 2MW1531-p and mutants thereof

### 1. Preparation of anti-CD47 control antibodies

Referring to the patents, the encoding genes of the light chain and heavy chain variable regions of the anti-CD47 control antibodies hu5F9 (Patent Publication US 9017675B2) and 1F8 (Patent Publication CN 110582515 A) were synthesized. The encoding genes of the light chain and heavy chain variable regions were respectively cloned into eukaryotic expression vectors containing the encoding genes of human light chain constant region and human IgG4 heavy chain constant region, to obtain expression plasmids of light chain and heavy chain, which were transformed into *Escherichia coli* for amplification, and then isolation was performed to obtain a large number of plasmids. The plasmids of the light chain and heavy chain of a control antibody were transferred into HEK293 cells for recombinant expression according to the operation instruction of the transfer reagent 293fectin. 5-6 days after transfection of cells, the culture supernatant was collected and purified by ProA affinity chromatography column to obtain a control antibody. In this application, the control antibody hu5F9 comprised a light chain variable region with an amino acid sequence set forth in SEQ ID NO: 35, and a heavy chain variable region with an amino acid sequence set forth in SEQ ID NO: 36. The control antibody 1F8 comprised a light chain variable region with an amino acid sequence set forth in SEQ ID NO: 37, and a heavy chain variable region with an amino acid sequence set forth in SEQ ID NO: 38.

### 2. Preparation of anti-human CD47/PD-L1 bispecific antibody 2MW1531-p and mutants thereof

The encoding gene of the variable region of the above hz140-H mutant with the amino acid sequence (SEQ ID NO: 23) and the encoding geneof the constant region of A chain of KIH with the amino acid sequence (SEQ ID NO: 39) were cloned in tandem into a transient expression vector, to construct an expression vector of the heavy chain hz140-H-A (amino acid sequence, SEQ ID NO: 40) of a bispecific antibody. The encoding gene (SEQ ID NO: 4) of the amino acid sequence (SEQ ID NO: 3) of the variable region of the above hz182-H and the encoding gene of the amino acid sequence (SEQ ID NO: 41) of B constant region of KIH were cloned in tandem into a transient expression vector, to construct an expression vector of the heavy chain hz182-H-B (amino acid sequence, SEQ ID NO: 42) of a bispecific antibody. Then the expression vectors of the two heavy chains and expression vectors of the common light chain of the two antibodies or a mutant thereof (amino acid sequences of the variable region of the light chain and mutant thereof were set forth in SEQ ID NOs: 1, 24, 25, 26, 27, 28, 29, 30, 31, 32, 33 and 34) were simultaneously transferred into an eukaryotic cell for expression, and then affinity purification was performed to obtain bispecific antibodies having common light chains in the form of "knobs-into-holes", named 2MW1531-p and 2MWT531-m1∼m11. Information of the mutants is shown in the table below. The mutation site in Fc of 2MW1531 hz140-H was: H315R. The mutated constant region (named hz140-H-knobs, A chain Fc) comprised an amino acid sequence set forth in SEQ ID NO: 39, and hz140-H-A comprised an amino acid sequence set forth in SEQ ID NO: 40. The mutation site in Fc of hz182-H was: K319E. The mutated constant region (named hz182-H-holes, B chain Fc) comprised an amino acid sequence set forth in SEQ ID NO: 41, and hz182-H-B comprised an amino acid sequence set forth in SEQ ID NO: 42. The pairing information of the light chain and heavy chain is shown in Table 8.

**Table 8. Pairing information of light chain and heavy chain of bispecific antibody 2MW1531-p and mutants thereof**

| Mutant | Number of amino acid sequence (SEQ ID NO: ) of bispecific antibody | | |
|---|---|---|---|
| | Light chain | Heavy chain A | Heavy chain B |
| 2MW1531-p | 1 | 40 | 42 |
| 2MW1531-ml | 24 | 40 | 42 |
| 2MW1531-m2 | 25 | 40 | 42 |
| 2MW1531-m3 | 26 | 40 | 42 |
| 2MW1531-m4 | 27 | 40 | 42 |
| 2MW1531-m5 | 28 | 40 | 42 |
| 2MW1531-m6 | 29 | 40 | 42 |
| 2MW1531-m7 | 30 | 40 | 42 |
| 2MW1531-m8 | 31 | 40 | 42 |
| 2MW1531-m9 | 32 | 40 | 42 |
| 2MW1531-m10 | 33 | 40 | 42 |
| 2MW1531-m11 | 34 | 40 | 42 |

### 3. Affinity analysis of CD47/PD-L1 bispecific antibodies

The affinity of 2MW1531-p and mutants thereof was determined using Fortebio. The affinity analysis results of each mutant to PD-L1 and CD47 are shown in Table 9 below.

**Table 9. Results of affinity assay of bispecific antibody 2MW1531-p and mutants thereof**

| | | KD (M) | kon(1/Ms) | kdis(1/s) |
|---|---|---|---|---|
| 2MW1531-p | CD47 | 8.56E-09 | 1.53E+05 | 1.31E-03 |
| | PD-L1 | 4.74E-10 | 3.10E+05 | 1.47E-04 |
| 2MW1531-m1 | CD47 | 2.25E-08 | 9.46E+04 | 2.13E-03 |
| | PD-L1 | 1.59E-09 | 2.43E+05 | 3.87E-04 |
| 2MW1531-m2 | CD47 | 8.10E-08 | 8.37E+04 | 6.78E-03 |
| | PD-L1 | 3.26E-09 | 2.13E+05 | 6.96E-04 |
| 2MW1531-m3 | CD47 | 6.57E-08 | 1.17E+05 | 7.69E-03 |
| | PD-L1 | 1.02E-09 | 1.59E+05 | 1.63E-04 |
| 2MW1531-m4 | CD47 | 2.65E-08 | 1.21E+05 | 3.21E-03 |
| | PD-L1 | 3.78E-09 | 1.87E+05 | 7.07E-04 |
| 2MW1531-m5 | CD47 | 1.08E-08 | 9.36E+04 | 1.01E-03 |
| | PD-L1 | 1.02E-09 | 3.47E+05 | 3.57E-04 |
| 2MW1531-m6 | CD47 | 8.45E-08 | 1.04E+05 | 8.79E-03 |
| | PD-L1 | 1.73E-09 | 2.87E+05 | 4.96E-04 |
| 2MW1531-m7 | CD47 | 6.21E-08 | 1.31E+05 | 8.14E-03 |
| | PD-L1 | 9.17E-10 | 2.30E+05 | 2.10E-04 |
| 2MW1531-m8 | CD47 | 1.07E-07 | 8.79E+04 | 9.37E-03 |
| | PD-L1 | 5.46E-10 | 2.45E+05 | 1.34E-04 |
| 2MW1531-m9 | CD47 | 7.96E-08 | 1.19E+05 | 9.47E-03 |
| | PD-L1 | 7.06E-10 | 3.85E+05 | 2.72E-04 |
| 2MW1531-m10 | CD47 | 5.48E-08 | 9.98E+04 | 5.47E-03 |
| | PD-L1 | 1.28E-09 | 2.88E+05 | 3.69E-04 |
| 2MW1531-m11 | CD47 | 7.49E-08 | 1.21E+05 | 9.07E-03 |
| | PD-L1 | 7.84E-10 | 3.02E+05 | 2.37E-04 |

### 4. Analysis of binding activity of CD47/PD-L1 bispecific antibody 2MW1531-p and mutants thereof to human red blood cells and tumor cells

The binding activity of 2MW1531-p and mutants thereof to human red blood cells and tumor cells (SKOV-3, which does not express PD-L1 when cultured *in vitro*) was analyzed by FACS. In the experiment of analyzing the binding to human red blood cells, the antibody was used at a concentration of 1000 nM; and in the experiment of analyzing the binding to tumor cells, the antibody was used at a concentration of 100 nM. The analysis results are shown in Table 10 below. 2MW1531-p and most of the mutants did not or weakly bind to human red blood cells, while exhibiting good binding activity to tumor cells.

**Table 10. Analysis results of binding activity of bispecific antibody 2MW1531-p and mutants thereof to human red blood cells and tumor cells**

| Mutant number | Binding to tumor cells (MFI) | Binding to human red blood cells (MFI) |
|---|---|---|
| 2MW1531-p | 35428.3 | 891.2 |
| 2MW1531-m1 | 33269.1 | 887.5 |
| 2MW1531-m2 | 29876.6 | 799.2 |
| 2MW1531-m3 | 34910.8 | 955.0 |
| 2MW1531-m4 | 31084.5 | 903.3 |
| 2MW1531-m5 | 30910.3 | 807.4 |
| 2MW1531-m6 | 24657.2 | 912.1 |
| 2MW1531-m7 | 28198.4 | 877.3 |
| 2MW1531-m8 | 28154.9 | 763.2 |
| 2MW1531-m9 | 37812.4 | 781.1 |
| 2MW1531-m10 | 26229.3 | 890.1 |
| 2MW1531-m11 | 28897.7 | 878.1 |
| human IgG4 | 4638.1 | 797.3 |

### Example 10: Analysis of the binding activity of anti-CD47/PD-L1 bispecific antibody 2MW1531 to cell surface antigens

### 1. Analysis of the binding activity of anti-human CD47/PD-L1 bispecific antibody 2MW1531 to MC38-hPD-L1/hCD47 cells

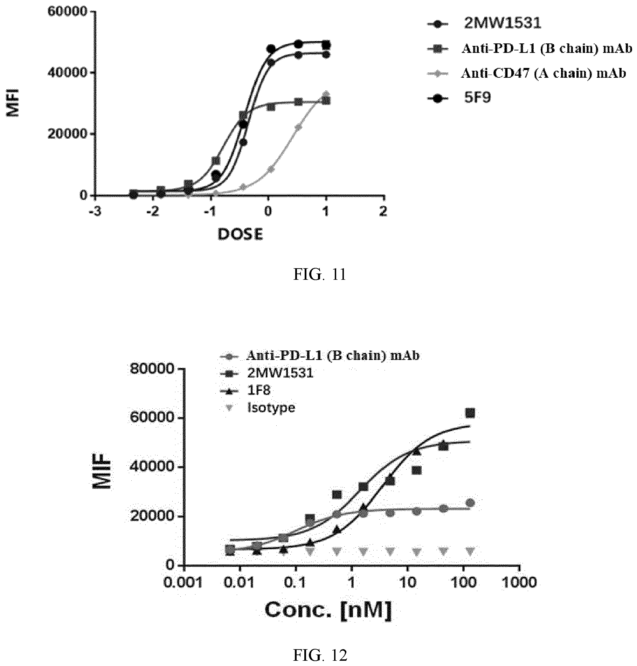

The binding activity of 2MW1531 (m9) to the mouse melanoma cell line MC38-hPD-L1/hCD47 which mouse PD-L1/CD47 gene was humanized was analyzed by flow cytometry. The results are shown in FIG. 11. The results show that in terms of the binding to the mouse melanoma cell line MC38-hPD-L1/hCD47 expressing human CD47 and human PD-L1: the anti-human CD47/PD-L1 bispecific antibody 2MW1531 had higher binding ability than the antibodies with high ability of specifically binding to CD47, including 5F9 control monoclonal antibody, anti-PD-L1 monoclonal antibody (light chain/heavy chain variable region comprising amino acid sequences set forth in SEQ ID NO: 1/SEQ ID NO: 3, and constant region comprising an amino acid sequence set forth in SEQ ID NO: 7/SEQ ID NO: 10); anti-CD47 monoclonal antibody (light chain/heavy chain variable region sequence set forth in SEQ ID NO: 5/SEQ ID NO: 8, and constant region sequence set forth in SEQ ID NO: 7/SEQ ID NO: 10). The results in FIG. 11 show that2MW1531 and the anti-CD47 sidearm monoclonal antibody and anti-PD-L sidearm monoclonal antibody thereof all had good binding activity to MC38-hPD-L1/hCD47 cells, and the binding signal of 2MW1531 was higher than that of the two homologous monoclonal antibodies, suggesting that the bispecific antibody had an additive or synergistic effect on the binding activity to cells.

### 2. Analysis of the binding activity of anti-human CD47/PD-L1 bispecific antibody 2MW1531 to A431 cells

The binding activity of 2MW1531(m9) to human skin squamous cell carcinoma cell line A431 expressing both CD47 and PD-L1 was analyzed by flow cytometry. The results are shown in FIG. 12. The experimental results show that in term of the binding to human skin squamous cell carcinoma cell line A431, the anti-human CD47/PD-L1 bispecific antibody 2MW1531 had higher binding ability than the anti-PD-L1 monoclonal antibody and the control anti-CD47 monoclonal antibody 1F8. The above results indicate that 2MW1531 had stronger binding activity than the homologous monospecific anti-PD-L1 monoclonal antibody and control anti-CD47 monoclonal antibody 1F8.

### Example 11: Binding activity of anti-human CD47/PD-L1 bispecific antibody 2MW1531 to red blood cell surface antigen

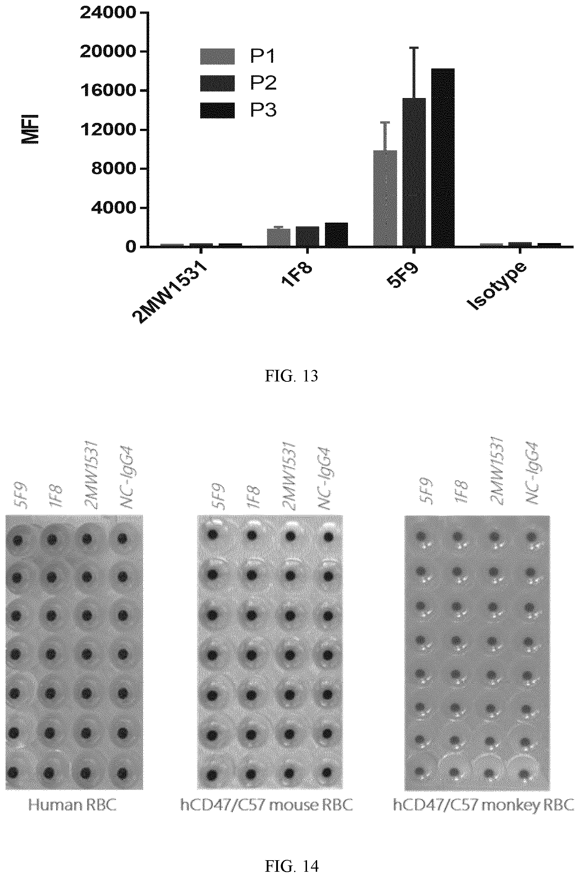

Human fresh anticoagulated peripheral blood was collected, and red blood cells were separated therefrom with human red blood cell separation kit. The red blood cells were divided into 5×10⁵cells/sample/100 µL, and added with serially diluted 2MW1531 (m7) at a final concentration of 200 nM. The cells were incubated on ice for 2 hours, washed twice with ice-cold PBS (containing 0.05% Tween); and added with FITC-labeled anti-human Fc secondary antibody (manufacturer, catalog number). The obtained cells were incubated on ice for 1 hour, washed twice with ice-cold PBS (containing 0.05% Tween), and resuspended in 200 µL of flow cytometry buffer. The suspension of cells was detected by flow cytometry. The test results are shown in FIG. 13. The results of flow cytometry in FIG. 13 show that there was no significant difference in the binding of 2MW1531 and the negative control antibody Isotype to human red blood cells, the anti-CD47 monoclonal antibody 1F8 had binding activity to human red blood cells, and the anti-CD47 monoclonal antibody 5F9 had a high binding ability to human red blood cells.

The results in FIG. 13 show that under the current conditions, 2MW1531 did not bind to human red blood cells, 1F8 weakly bound to human red blood cells, and 5F9 strongly bound to human red blood cells.

### Example 12: In vitro RBC agglutination test of anti-human CD47/PD-L1 bispecific antibody 2MW1531

Fresh anticoagulated peripheral blood of human, CD47 humanized C57 mice (hCD47/C57), and cynomolgus monkeys was collected, and red blood cells were separated therefrom with a red blood cell separation kit. The red blood cells were counted, adjusted for cell density, and added to a U-bottom 96-well cell plate at 1×10⁷ cells/well/100 µL. 2MW1531 (m11) was diluted with PBS from 800 µg/ml for 7 gradients by 4-fold dilution, and the diluted solutions were added in an equal volume (100 µL) to each cell well. The resulting mixture was mixed gently, so that the final concentration of cells was 0.5×10⁷ cells/well/200 µL, and the final working concentration of the antibody to be tested started at 400 µg/ml. The cell plate added with the samples was put in a 37°C cell incubator and incubated for 4 hours. The coagulation phenomenon was observed with naked eyes, and the judging standard was as follows: if the red blood cells were deposited on the bottom and the supernatant was clear, it was no agglutination; if the supernatant was clear but the red blood cells were diffused at the bottom of the culture plate, it was agglutination; and if the supernatant was turbid and reddish brown, it was hemolysis.

The results are shown in FIG. 14. The experimental results show that in the agglutination test of human red blood cells, CD47-humanized C57 mouse red blood cells, and cynomolgus monkey red blood cells, the anti-CD47 monoclonal antibodies 5F9 and 1F8 caused no red blood cells agglutination; for 2MW1531 and NC-IgG4, all the red blood cells were concentrated at the bottom of the microwell plate without reticular agglutination. The above results show that 2MW1531 caused no red blood cells aggregation or hemolysis under the current concentration conditions.

### Example 13: In vivo anti-tumor activity of anti-human CD47/PD-L1 bispecific antibody 2MW1531

### 1. Determination of anti-tumor efficacy of 2MW1531 on hPD-L1/hCD47/hSIRPα humanized mouse subcutaneously homografted MC38-hPDL1 mouse colon cancer tumor model

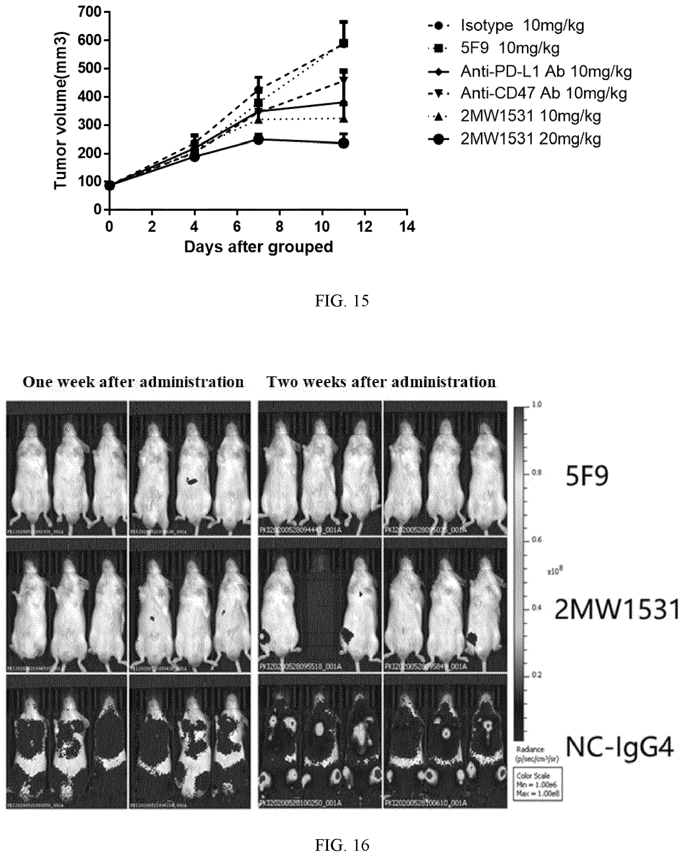

Murine colon cancer tumor cell hPDL1/CD47-MC38 humanized with PD-L1 and CD47 was subcutaneously inoculated into the lateral anterior flank of female hPD-L1/hCD47/hSIRPα transgenic mice (hPD-L1/hCD47/hSIRPα transgenic mice derived from C57). When the tumor grew to about 50-100 mm³, the mice were grouped and intraperitoneally administered with 2MW1531 (m7) or isotype control IgG at an amount of 20 mg/kg and 10 mg/kg, twice a week. The tumor volume and body weight were measured during each administration, and the relationship between the changes in the body weight and tumor volume of the tumor-bearing mice and the administration time was recorded. The results are shown in FIG. 15. In FIG. 15, the results of the *in vivo* test in colon cancer tumor animal model show that, under the same dose (10 mg/kg), 2MW1531 had stronger inhibitory effect on tumor volume than the anti-CD47 monoclonal antibody, and had equivalent or slightly stronger effect than the anti-PD-L1 monoclonal antibody. Under an increased dose (20 mg/kg), 2MW1531 had further significantly improved inhibitory effect on tumor volume. The above results show that the bispecific antibody 2MW1531 effectively inhibited tumor growth in a dose-dependent manner, and had stronger activity than the anti-CD47 and anti-PD-L1 monoclonal antibodies.

### 2. Determination of antitumor efficacy of 2MW1531 on Raji-Luc blood tumor model in nude mice

In order to determine the effect of the anti-CD47 binding arm of the bispecific antibody 2MW1531 on tumor therapy, Raji-Luc cells that did not express PD-L1 were further used for *in vivo* animal experiment. The antitumor efficacy of 2MW1531 (m11) was analyzed in immunodeficient mice (NCG) tail vein xenograft Raji-Luc hematological tumor model. First, hematological tumor cell Raji-Luc was xenografted into female immunodeficient mice (NCG) through the tail vein. Then the proliferation of hematological tumor cells was indicated by measuring the fluorescence signal intensity in the mouse using a mouse *in vivo* imaging system. The results are shown in FIGs. 16 and 9. The *in vivo* imaging in FIG. 16 shows that in the Raji tumor cell transplantation mouse model that did not express PD-L1, the anti-human CD47/PD-L1 bispecific antibody 2MW1531 had a significant inhibitory effect on Raji tumor cell growth, and had a comparable inhibitory effect on the proliferation of Raji tumor cells to that of the anti-CD47 monoclonal antibody 5F9. The quantitative analysis of radiation signals in FIG. 9 shows that after intravenously inoculated with Raji tumor cells, the mice were intraperitoneally injected with 2MW1531 twice a week, and 2MW1531 significantly inhibited the growth of tumor cells, and had an equivalent therapeutic effect to that of the anti-CD47 monoclonal antibody 5F9. The above results show that Raji-Luc cells inoculated in NCG mice can proliferate rapidly, and the administration of 2MW1531 for treatment can inhibit and eliminate the tumor cells in mice, suggesting that when there is no PD-L1 target on tumor cells, 2MW1531 can block the CD47 signaling pathway through a single sidearm, thereby promoting the phagocytosis of macrophages and exerting anti-tumor effects.

Although specific embodiments of the present disclosure have been described in detail, those skilled in the art will understand that based on all the teachings that have been disclosed, various modifications and replacements may be made to those details, and these changes are all within the scope of the present disclosure. The full scope of the present disclosure is given by the claims and any equivalents thereof.

In the description of this specification, references to the terms "one embodiment," "some embodiments," "exemplary embodiments," "example," "specific examples," or "some examples" are intended to mean that the combination of the specific features, structures, materials, or characteristics described by the embodiment or example is included in at least one embodiment or example of the present disclosure. In this specification, schematic descriptions of the above terms do not necessarily refer to the same embodiment or example. Furthermore, the specific features, structures, materials or characteristics described may be combined in any suitable manner in any one or more embodiments or examples.

## Claims

1. A humanized anti-human CD47 monoclonal antibody or a fragment thereof, comprising:
a heavy chain CDR1 comprising X₁YX₂MX₃, wherein X₁ is selected from the group consisting of N and S, X₂ is selected from the group consisting of V and A, and X₃ is selected from the group consisting of H and S;
a heavy chain CDR2 comprising YINPX₄NX₅X₆IKYNEKFX₇G, wherein X₄ is selected from the group consisting of Y and G, X₅ is selected from the group consisting of D and E, X₆ is selected from the group consisting of G and A, and X₇ is selected from the group consisting of T and Q; and
a heavy chain CDR3 comprising EGDFYANYGRLGFX₈Y, wherein X₈ is selected from the group consisting of A and D; and
a light chain CDR1 comprising RASQDIX₉NYLN, wherein X₉ is selected from the group consisting of S and T;
a light chain CDR2 comprising YTSRLX₁₀S, wherein X₁₀ is selected from the group consisting of H, Q and S; and
a light chain CDR3 comprising QQGX₁₁X₁₂X₁₃PX₁₄T, wherein X₁₁ is selected from the group consisting of D and A, X₁₂ is selected from the group consisting of T and G, X₁₃ is selected from the group consisting of F, R, Y, K, S, E and N, and X₁₄ is selected from the group consisting of Y and R.

2. The humanized anti-human CD47 monoclonal antibody or fragment thereof according to claim 1, which is derived from a germline template selected from the group consisting of IGKV1-33^{∗}01|IGKJ4^{∗}02 and IGHV1-3^{∗}01|IGHJ1^{∗}01.

3. The humanized anti-human CD47 monoclonal antibody or fragment thereof according to claim 1 or 2, wherein the CDRs and/or FR regions comprise a point mutation.

4. The humanized anti-human CD47 monoclonal antibody or fragment thereof according to claim 1, wherein the heavy chain CDR1 comprises a sequence set forth in SEQ ID NO: 76, 77, 78 or 87;
the heavy chain CDR2 comprises a sequence set forth in SEQ ID NO: 79, 80, 81, 82, 83 or 84; and
the heavy chain CDR3 comprises a sequence set forth in SEQ ID NO: 85 or 86.

5. The humanized anti-human CD47 monoclonal antibody or fragment thereof according to claim 1, wherein
the light chain CDR1 comprises a sequence set forth in SEQ ID NO: 46 or 47;
the light chain CDR2 comprises a sequence set forth in SEQ ID NO: 50, 51 or 52; and
the heavy chain CDR3 comprises a sequence set forth in SEQ ID NO: 56, 57, 58, 59, 60, 61, 62, 63, 64, 65 or 68.

6. The humanized anti-human CD47 monoclonal antibody or fragment thereof according to any one of claims 1-5, wherein the antibody or antigen-binding fragment thereof comprises a heavy chain variable region set forth in SEQ ID NO: 8, 11, 12, 13, 14, 15, 16, 17, 18, 19, 20, 21, 22 or 23, and a light chain variable region set forth in SEQ ID NO: 5.

7. The humanized anti-human CD47 monoclonal antibody or fragment thereof according to any one of claims 1-6, wherein the monoclonal antibody is an antibody derived from an original monoclonal antibody by CDRs transplantation, affinity maturation, point mutation and chemical modification, wherein the chemical modification is selected from the group consisting of glycosylation, acetylation, pegylation, phosphorylation, amidation, protease cleavage, linking with cellular ligands or effector molecules, and protection and/or blocking of an active reactive group.

8. The humanized anti-human CD47 monoclonal antibody or fragment thereof according to any one of claims 1-7, wherein the fragment is a Fab, Fab', F(ab')2, Fv, scFv or dAb.

9. A multispecific antibody comprising at least one specific binding region for human CD47, wherein the specific binding region for human CD47 is derived from the humanized anti-human CD47 monoclonal antibody or fragment thereof according to any one of claims 1-8.

10. The multispecific antibody according to claim 9, comprising multiple specific binding regions for different epitopes of human CD47.

11. The multispecific antibody according to any one of claims 9-10, further comprising a specific binding region which does not target human CD47.

12. The multispecific antibody according to any one of claims 9-11, wherein the multispecific antibody is a bispecific antibody, a trispecific antibody or a tetraspecific antibody.

13. Abispecific antibody targeting PD-L1 and CD47 or an antigen-binding fragment thereof, comprising two heavy chains and two light chains, wherein a first heavy chain variable region is derived from a heavy chain variable region of an anti-CD47 monoclonal antibody, which forms a CD47 binding region with a light chain variable region; and a second heavy chain variable region is derived from a heavy chain variable region of an anti-PD-L1 monoclonal antibody, which forms a PD-L1 binding region with a light chain variable region;
wherein the light chain variable regions of the two light chains are the same, and the light chain variable region is derived from the light chain of the anti-CD47 monoclonal antibody and the light chain of the anti-PD-L1 monoclonal antibody, and
wherein a first binding region specifically binding to CD47 is formed by the light chain variable region and the first heavy chain variable region, and a second binding region specifically binding to CD47PD-L1 is formed by the light chain variable region and the second heavy chain variable region; and
wherein the anti-CD47 monoclonal antibody is the humanized anti-human CD47 monoclonal antibody or fragment thereof according to any one of claims 1-8.

14. The bispecific antibody targeting PD-L1 and CD47 or antigen-binding fragment thereof according to claim 13, wherein the anti-PD-L1 monoclonal antibody comprises a heavy chain variable region set forth in SEQ ID NO: 3 and a light chain variable region set forth in SEQ ID NO: 1.

15. The bispecific antibody targeting PD-L1 and CD47 or antigen-binding fragment thereof according to claim 13, wherein the second heavy chain variable region comprises a CDR1 set forth in SEQ ID NO: 46 and a CDR3 set forth in SEQ ID NO: 61, 62, 63, 70, 71 or 72.

16. The bispecific antibody targeting PD-L1 and CD47 or antigen-binding fragment thereof according to claim 13, wherein the light chain comprises a sequence set forth in SEQ ID NO: 1, 24, 25, 26, 27, 28, 29, 30, 31, 32, 33 or 34.

17. The bispecific antibody targeting PD-L1 and CD47 or antigen-binding fragment thereof according to any one of claims 13-16, including but not limited to a complete antibody or F(ab')₂, wherein the complete antibody is selected from the group consisting of IgG1, IgG2a, IgG2b, IgG3 and IgG4 type antibodies.

18. The bispecific antibody targeting PD-L1 and CD47 or antigen-binding fragment thereof according to claim 13, wherein the complete antibody is selected from the group consisting of IgG1 and IgG4 type antibodies.

19. The bispecific antibody targeting PD-L1 and CD47 or antigen-binding fragment thereof according to any one of claims 13-18, wherein the two heavy chains comprise Fc fragments containing a mutation for "knobs-into-holes".

20. The bispecific antibody targeting PD-L1 and CD47 or antigen-binding fragment thereof according to claim 13, wherein the first heavy chain comprises an Fc fragment containing H315R mutation, the second heavy chain comprises an Fc fragment containing K319E mutation, and amino acid mutation positions in the Fc fragment are in Eu Kabat numbering system.

21. A polynucleotide encoding the humanized anti-human CD47 monoclonal antibody or fragment thereof according to any one of claims 1-8, or the multispecific antibody according to any one of claims 9-12, or the bispecific antibody targeting PD-L1 and CD47 or antigen-binding fragment thereof according to any one of claims 13-20.

22. A vector comprising the polynucleotide according to claim 21.

23. A host cell comprising the polynucleotide according to claim 21 or the vector according to claim 22.

24. A method for preparing an antibody or an antigen-binding fragment thereof, comprising the following steps:
(1) culturing the host cell according to claim 23 under a condition suitable for expressing a bispecific antibody or an antigen-binding fragment thereof; and
(2) isolating and purifying the bispecific antibody or fragment thereof from cell culture.

25. A composition comprising at least one selected from the group consisting of the humanized anti-human CD47 monoclonal antibody or fragment thereof according to any one of claims 1-8, the multispecific antibody according to any one of claims 9-12, the bispecific antibody targeting PD-L1 and CD47 or antigen-binding fragment thereof according to any one of claims 13-20, the polynucleotide according to claim 21, the vector according to claim 22, and the host cell according to claim 23.

26. Use of the humanized anti-human CD47 monoclonal antibody or fragment thereof according to any one of claims 1-8, the multispecific antibody according to any one of claims 9-12, the bispecific antibody targeting PD-L1 and CD47 or antigen-binding fragment thereof according to any one of claims 13-20, the polynucleotide according to claim 21 and the composition according to claim 25 in the manufacture of a medicament for treating a tumor and/or improving immune response in the body.

27. The use according to claim 26, wherein the tumor is selected from the group consisting of melanoma, lung cancer, kidney cancer, Hodgkin's lymphoma, head and neck squamous cell carcinoma, urothelial carcinoma, acute and chronic myeloid leukemia, acute lymphoblastic leukemia, non-Hodgkin's lymphoma, bladder cancer, ovarian cancer, breast cancer, colorectal cancer, prostate cancer, kidney cancer, and multiple myeloma.

28. A method for treating a subject suffering from a tumor, comprising administering a therapeutically effective amount of the composition according to claim 25 to the subject in need thereof.

29. The method according to claim 28, wherein the tumor is selected from the group consisting of melanoma, lung cancer, kidney cancer, Hodgkin's lymphoma, head and neck squamous cell carcinoma, urothelial carcinoma, acute and chronic myeloid leukemia, acute lymphoblastic leukemia, non-Hodgkin's lymphoma, bladder cancer, ovarian cancer, breast cancer, colorectal cancer, prostate cancer, kidney cancer, and multiple myeloma.
